# EUROPÄISCHE PATENTSCHRIFT

(11) **EP 4 065 544 B1**
(45) Veröffentlichungstag und Bekanntmachung des Hinweises auf die Patenterteilung: **11.02.2026**
(21) Anmeldenummer: 20808133.1
(22) Anmeldetag: 23.11.2020
(51) Int. Cl.: C07C 51/50, C07C 57/04, C07C 67/62, C07C 69/54

(54) **LAGER- UND TRANSPORTSTABILISATOREN FÜR POLYMERISATIONSFÄHIGE VERBINDUNGEN**
STORAGE AND TRANSPORT STABILIZERS FOR POLYMERIZABLE COMPOUNDS
STABILISATEURS DE STOCKAGE ET DE TRANSPORT POUR COMPOSÉS POLYMÉRISABLES

(30) Priorität: 28.11.2019 EP 19212189
(43) Veröffentlichungstag der Anmeldung: 05.10.2022
(73) Patentinhaber: BASF SE, 67056 Ludwigshafen am Rhein (DE)
(72) Erfinder: HAREMZA, Sylke, 69151 Neckargemünd (DE); MARTIN, Friedrich-Georg, 67056 Ludwigshafen am Rhein (DE); KREMZOW-GRAW, Doris, 67056 Ludwigshafen am Rhein (DE); SURE, Rebecca, 67056 Ludwigshafen am Rhein (DE); ROMEIS, Juergen, 67056 Ludwigshafen am Rhein (DE); SCHELKLE, Korwin, Mumbai 400705 (IN); FLEISCHHAKER, Friederike, 67056 Ludwigshafen am Rhein (DE); GORGES, Jan, Niclas, 67056 Ludwigshafen am Rhein (DE)
(74) Vertreter: BASF IP Association
(86) Internationale Anmeldenummer: PCT/EP2020/083014
(87) Internationale Veröffentlichungsnummer: WO 2021/105038

(56) Entgegenhaltungen:
- WO-A2-2007/063031

## Beschreibung

Chemische Verbindungen, die eine oder mehrere ethylenisch ungesättigte Gruppen aufweisen, haben eine ausgeprägte Neigung zur radikalischen Polymerisation. Nachfolgend werden solche Verbindungen daher auch als polymerisationsfähige Verbindungen bezeichnet. Aufgrund ihrer Neigung zur radikalischen Polymerisation werden diese Verbindungen als Monomere zur Herstellung von Polymeren, z.B. durch gewollte radikalische Polymerisation, verwendet. Die ausgeprägte Neigung zur radikalischen Polymerisation dieser Verbindungen ist jedoch insofern von Nachteil, da es sowohl bei der Lagerung und beim Transport als auch bei der chemischen und/oder physikalischen Bearbeitung, z.B. durch Destillation oder Rektifikation, insbesondere unter der Einwirkung von Energie, z.B. Wärme und/oder Licht, zur unerwünschten, spontanen radikalischen Polymerisation kommen kann. Derartig unkontrollierte Polymerisationen können zur allmählichen Bildung von Polymerbelägen, z.B. auf erwärmten Oberflächen führen, was eine Entfernung der Polymerbeläge notwendig macht und damit oft zu einer Reduktion der Betriebszeiten führt, oder sogar explosionsartig verlaufen.

Es ist daher üblich, ethylenisch ungesättigten Verbindungen, die eine Neigung zur radikalischen Polymerisation haben, beziehungsweise Gemischen, die solche Verbindungen enthalten, sowohl bei der Lagerung und beim Transport als auch bei der chemischen und/oder physikalischen Bearbeitung Substanzen zuzusetzen, die eine unerwünschte, spontane radikalische Polymerisation unterbinden, beziehungsweise verlangsamen. Derartige Substanzen werden im Allgemeinen und im Folgenden als Polymerisationsinhibitoren oder Polymerisationsstabilisatoren bezeichnet.

Polymerisationsstabilisatoren können als chemische Einzelverbindungen oder als Gemische von Verbindungen eingesetzt werden. Je nach Einsatzgebiet des Polymerisationsstabilisators sind bestimmte Anforderungen an diesen gerichtet. Für die Eignung eines Polymerisationsstabilisators als Transport- und/oder Lagerstabilisator von ethylenisch ungesättigten Verbindungen ist es z. B. wichtig, dass die Effizienz des Polymerisationsstabilisators, d.h. das Ausmaß der Polymerisationsstabilisierung bzw. Polymerisationsinhibierung, reguliert werden kann. Unter den Bedingungen der Lagerung und/oder dem Transport der ethylenisch ungesättigten Verbindungen soll der Polymerisationsstabilisator die unerwünschte, spontane radikalische Polymerisation ausreichend unterbinden, beziehungsweise verlangsamen, wohingegen die gewollte radikalische Polymerisation der ethylenisch ungesättigten Verbindungen unter entsprechenden Polymerisationsbedingungen möglich sein soll, ohne die Notwendigkeit, den bei der Lagerung und/oder dem Transport verwendeten Polymerisationsstabilisator zuvor abtrennen zu müssen. Wird der bei der Lagerung und/oder Transport verwendete Stabilisator bei der gewollten Polymerisation nicht abgetrennt, ist es wichtig, dass dieser während der gewollten Polymerisation diese nicht nachteilig beeinflusst, bspw. ungewollt als Regler wirkt (zu Polymerisationsregler siehe beispielsweise Ullmann's Encyclopedia of Industrial Chemistry, Polymerization Processes, 1. Fundamentals, 6 Free-Radical Polymerization, DOI 10.1002/14356007.21_305.pub3 oder Ullmann's Encyclopedia of Industrial Chemistry, Polyacrylates, 4.1.1 Starting Materials, DOI 10.1002/14356007.a21_157.pub2).

Gemessen am weltweiten Produktionsvolumen ist Acrylsäure sicherlich eine der bedeutsamsten ethylenisch ungesättigten Verbindungen. Standardmäßig wird Acrylsäure während der Lagerung und/oder dem Transport mit 180 bis 220 Gew.-ppm Hydrochinonmonomethylether (MEHQ), bezogen auf die Menge Acrylsäure, gegen unerwünschte, spontane radikalische Polymerisation stabilisiert. Für eine ausreichende Stabilisierung der Acrylsäure gegen unerwünschte, spontane radikalische Polymerisation mittels MEHQ ist es notwendig, dass Sauerstoff in ausreichenden Mengen in der Acrylsäure gelöst ist. Ausreichende Mengen von Sauerstoff sind in der Regel dann in der Acrylsäure gelöst, wenn Acrylsäure unter einer Atmosphäre mit 5 bis 21 vol.% Sauerstoff gelagert und/oder transportiert wird. Die Stabilisierung von Acrylsäure durch MEHQ bei Lagerung und/oder Transport ist beispielsweise in Acrylic Acid, A Summary of Safety and Handling, 4th Edition 2013, 6.1 Instability and Reactivity - Polymerisation, und 7.1. Bulk Storage Facilities and Accessories - General Considerations beschrieben. Bei der gewollten Polymerisation von Acrylsäure, beispielsweise bei der Herstellung von Polyacrylaten, wird der in der Acrylsäure gelöste Sauerstoffgehalt verringert, wodurch die Effizienz von MEHQ zur Polymerisationsstabilisierung derart reduziert wird, dass Acrylsäure in Gegenwart von MEHQ polymerisiert werden kann.

Neben der Verwendung als Polymerisationsstabilisator bei der Lagerung und/oder Transport von Acrylsäure findet MEHQ auch Verwendung als Polymerisationsstabilisator bei der Lagerung und/oder Transport von Methacrylsäure, Acrylsäureestern, Methacrylsäureestern oder Gemischen, die eine oder mehr der zuvor genannten Verbindungen enthalten. Acrylsäure oder Methacrylsäure werden im Folgenden als (Meth)-acrylsäure bezeichnet, deren Ester werden im Folgenden als (Meth)acrylsäureester bezeichnet.

Aufgrund der breiten Verwendung stellt MEHQ einen der wichtigsten Lager- und/oder Transportstabilisatoren für polymerisationsfähige Verbindungen im Allgemeinen und für (Meth)acrylsäure, bzw. (Meth)acrylsäureester im Speziellen dar.

Die Lagerung von polymerisationsfähigen Verbindungen erfolgt in dafür geeigneten, fest installierten Gebinden wie Lagertanks oder Lagerbehälter (siehe beispielsweise Acrylic Acid, A Summary of Safety and Handling, 4th Edition 2013, 7 Bulk Storage Facilities and Accessories). Es ist bevorzugt, dass sich bei der Lagerung die polymerisationsfähigen Verbindungen für 1 Minute oder mehr, weiter bevorzugt für 10 Minuten oder mehr und besonders bevorzugt für 60 Minuten oder mehr in den jeweiligen Gebinden befinden. Obwohl die Dauer der Lagerung unter entsprechenden Bedingungen theoretisch unbeschränkt ist, wird die Lagerungsdauer in der Regel aus wirtschaftlichen Gründen auf ein Minimum reduziert. Es ist bevorzugt, dass sich bei der Lagerung die polymerisationsfähigen Verbindungen für maximal 180 Tage, weiter bevorzugt für maximal 90 Tage und besonders bevorzugt für maximal 30 Tage in den jeweiligen Gebinden befinden. Besonders bevorzugte Bereiche ergeben sich aus der beliebigen Kombination der zuvor genannten unteren und oberen Grenzen.

Der Transport von polymerisationsfähigen Verbindungen erfolgt üblicherweise in dafür geeigneten, transportablen Gebinden wie Tanks oder Behälter per Schiff, Eisenbahnwagen und/oder Lastwagen (siehe beispielsweise Acrylic Acid, A Summary of Safety and Handling, 4th Edition 2013, 9 Safe Transport of Acrylic Acid). Natürlich können die transportablen Gebinde auch fest auf dem entsprechenden Transportmittel installiert sein, beispielsweise Schifftanks oder Bahnkesselwagen. Natürlich ist es auch möglich, dass die Gebinde für eine gewisse Zeit an einer bestimmten Stelle gelagert werden, bevor diese an eine andere Stelle transportiert werden. Der Transport von polymerisationsfähigen Verbindungen kann auch in Rohrleitungen oder Schläuchen erfolgen, beispielsweise nach Aufreinigung der polymerisationsfähigen Verbindungen zum Lagertank, bei der Verladung vom Lagertank in ein transportables Gebinde und/oder bei der Verladung von einem transportablen Gebinde in ein anderes. Es ist bevorzugt, dass sich beim Transport die polymerisationsfähigen Verbindungen für eine Dauer von 10 Sekunden oder mehr, weiter bevorzugt für 1 Minute oder mehr, besonders bevorzugt für 10 Minuten oder mehr und besonders bevorzugt für 60 Minuten oder mehr in den jeweiligen Gebinden befinden. Obwohl die Dauer unter entsprechenden Bedingungen theoretisch unbeschränkt ist, wird die Dauer in der Regel aus wirtschaftlichen und sicherheitstechnischen Gründen auf ein Minimum reduziert. Es ist bevorzugt, dass sich beim Transport die polymerisationsfähigen Verbindungen für maximal 180 Tage, weiter bevorzugt für maximal 90 Tage und besonders bevorzugt für maximal 30 Tage in den jeweiligen Gebinden befinden. Besonders bevorzugte Bereiche ergeben sich aus der beliebigen Kombination der zuvor genannten unteren und oberen Grenzen.

Um die Abhängigkeit von MEHQ zu reduzieren, war es die Aufgabe der vorliegenden Erfindung, Mischungen zur Verfügung zu stellen, die (Meth)acrylsäure und/oder (Meth)acrylsäureester mit C₁- bis C₈-Alkyl und einen oder mehrere Lager- und/oder Transportstabilisatoren enthalten. Die Lager - und/oder Transportstabilisatoren sollen eine ausreichende Stabilisierung der (Meth)acrylsäure und/oder (Meth)acrylsäureester mit C₁- bis C₈-Alkyl gegen ungewollte radikalische Polymerisation gewährleisten. Weiterhin wäre es von Vorteil, dass bei der gewollten radikalischen Polymerisation keine Notwendigkeit besteht, die Lager- und/oder Transportstabilisatoren vor der Polymerisation aus der Mischung abzutrennen. Die Lager- und oder Transportstabilisatoren sollten daher bei der gewollten radikalischen Polymerisation nicht als Regler und/oder Inhibitoren wirken.

Im Rahmen der vorliegenden Erfindung ist eine Verbindung dann als Lager- und/oder Transportstabilisator von (Meth)acrylsäure und/oder (Meth)acrylsäureester mit C₁- bis C₈-Alkyl geeignet und wirkt nicht als Regler und/oder Inhibitor bei der gewollten radikalischen Polymerisation, wenn diese Verbindung unter vergleichbaren Bedingungen
a) die spontane radikalische Polymerisation von flüssiger Acrylsäure, die unter einer Atmosphäre mit 5 bis 30 vol. % Sauerstoff gelagert wird mit einer Effizienz von 75 oder mehr Prozent bezogen auf die Effizienz einer äquimolaren Menge MEHQ inhibiert
   und
b) die radikalische Polymerisation von flüssiger Acrylsäure, die unter einer Atmosphäre mit 96 bis 100 vol.% Stickstoff gelagert wird und mit 1000 bis 15000 Gew. ppm Polymerisationsstarter versetzt ist, mit einer Effizienz von 130 oder weniger Prozent, bezogen auf die Effizienz einer äquimolaren Menge MEHQ inhibiert und nach der Polymerisation vergleichbare bzw. ähnliche Polymere erhalten werden. Bevorzugt beträgt der Stickstoffgehalt der Atmosphäre 99 bis 100 vol. %.

MEHQ wird in a) und b) jeweils in Mengen von 10 bis 250 Gew.-ppm eingesetzt, bevorzugt in einer Menge von 35 bis 45 Gew.-ppm. Angaben in Gew.-ppm beziehen sich auf die Menge an eingesetzter flüssiger Acrylsäure.

Vergleichbare bzw. ähnliche Polymere werden dann erhalten, wenn die Polymere im Wesentlichen identische mittlere Gewichtsmittel (gemessen nach der gleichen GPC-Methode) aufweisen. Im wesentlich identische Gewichtsmittel liegen dann vor, wenn die Gewichtsmittel um 20 Prozent oder weniger auseinander liegen.

Als Polymerisationsstarter eignen sich die dem Fachmann bekannten Polymerisationsstarter, die üblicherweise zur Polymerisation von (Meth)acrylsäure eingesetzt werden (siehe beispielsweise The Chemistry of Radical Polymerisation, 2nd Edition, 2005, Seite 49 bis 166).

WO 2007/063031 A1 offenbart Mischungen, enthaltend eine aromatischen Heterocyclus und eine polymerisationsfähige Verbindung. Als aromatische Heterocyclen werden auch 3-Pyrazole eingesetzt.

Die Aufgabe wird gelöst durch eine Mischung enthaltend eine oder mehrere Verbindungen der allgemeinen Formel (II) 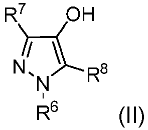 wobei
R⁶ H oder C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst,
R⁷ H, C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst, oder Alkanoyl mit C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst, und
R⁸ H oder C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst,
und (Meth)acrylsäure und/oder (Meth)acrylsäureester mit C₁- bis C₈-Alkyl, wobei C₁-bis C₈-Alkyl gerade oder verzweigte C₁- bis C₈-Alkylgruppen oder cyclische C₃- bis C₈-Alkylgruppen umfasst.

C₁- bis C₈-Alkyl umfasst gerade oder verzweigte C₁- bis C₈-Alkylgruppen oder cyclische C₃- bis C₈-Alkylgruppen. Bei C₁- bis C₈-Alkyl handelt es sich beispielsweise um Methyl, Ethyl, n-Propyl, iso-Propyl, n-Butyl, sec-Butyl, iso-Butyl, tert.-Butyl, n-Pentyl, 2-Methylbutyl, 3-Methylbutyl, Cyclopentyl, n-Hexyl, Cyclohexyl, 2-Methylpentyl, 3-Methylpentyl, 4-Methylpentyl, 3,3-Dimethylbutyl, 2-Ethylbutyl, n-Heptyl, 2-Methylhexyl, 2-Ethylpentyl, 4-Methylcyclohexyl, n-Octyl, iso-Octyl oder 2-Ethylhexyl.

Ein weiterer Gegenstand der vorliegenden Erfindung ist die Verwendung einer oder mehrerer Verbindungen der allgemeinen Formel (II) als Lager- und/oder Transportstabilisator von (Meth)acrylsäure und/oder (Meth)acrylsäureester mit C₁- bis C₈-Alkyl.

Weiterhin ist ein Verfahren zur Stabilisierung von (Meth)acrylsäure und/oder (Meth)acrylsäureester mit C₁- bis C₈-Alkyl bei der Lagerung und/oder Transport unter Verwendung einer oder mehrerer Verbindungen der allgemeinen Formel (II) Gegenstand der vorliegenden Erfindung.

Ebenfalls Gegenstand der Erfindung ist ein Verfahren zur Polymerisation von (Meth)acrylsäure und/oder (Meth)acrylsäureester mit C₁- bis C₈-Alkyl in Gegenwart einer oder mehrerer Verbindungen der allgemeinen Formel (II).

Die erfindungsgemäße Mischung ist unter Standardbedingungen flüssig.

### Beschreibung:

In Verbindungen der allgemeinen Formel (II)
ist 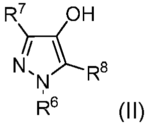
R⁶ H oder C₁- bis C₆-Alkyl, wobei C₁- bis C₆-Alkyl gerade oder verzweigte C₁- bis C₆-Alkylgruppen oder cyclische C₃- bis C₆-Alkylgruppen umfasst,
R⁷ H, C₁- bis C₆-Alkyl, wobei C₁- bis C₆-Alkyl gerade oder verzweigte C₁- bis C₆-Alkylgruppen oder cyclische C₃- bis C₆-Alkylgruppen umfasst, Alkanoyl mit C₁- bis C₆-Alkyl, wobei C₁- bis C₆-Alkyl gerade oder verzweigte C₁- bis C₆-Alkylgruppen oder cyclische C₃- bis C₆-Alkylgruppen umfasst, und
R⁸ H oder C₁- bis C₆-Alkyl, wobei C₁- bis C₆-Alkyl gerade oder verzweigte C₁- bis C₆-Alkylgruppen oder cyclische C₃- bis C₆-Alkylgruppen umfasst.

Ebenfalls offenbart ist eine Mischung enthaltend eine oder mehrere Verbindungen der allgemeinen Formel (II) und (Meth)acrylsäure und/oder (Meth)acrylsäureester mit C₁-bis C₈-Alkyl.

Bei (Meth)acrylsäureester mit C₁- bis C₈-Alkyl. handelt es sich bevorzugt um Methyl(meth)acrylat, Ethyl(meth)acrylat, n-Butyl(meth)acrylat, 2-Ethylhexyl(meth)acrylat oder um eine Mischung aus zwei oder mehr der zuvor genannten (Meth)acrylsäureester.

Die Herstellung von Verbindungen der allgemeinen Formel (II) ist dem Fachmann bekannt oder erschließt sich ihm aus seinem allgemeinen Fachwissen.

Die Gesamtmenge der Verbindungen der allgemeinen Formel (II) in der Mischung beträgt bevorzugt 0,1 bis 1000 Gew.-ppm bezogen auf das Gesamtgewicht der in der Mischung enthaltenen Menge an (Meth)acrylsäure und (Meth)acrylsäureester mit C₁-bis C₈-Alkyl. Besonders bevorzugt beträgt die Gesamtmenge der Verbindungen der allgemeinen Formel (II) in der Mischung 1 bis 900 Gew.-ppm, ganz besonders bevorzugt 10 bis 800 Gew.-ppm und insbesondere 10 bis 500 Gew.-ppm, jeweils bezogen auf das Gesamtgewicht der in der Mischung enthaltenen Menge an (Meth)acrylsäure und (Meth)acrylsäureester mit C₁- bis C₈-Alkyl.

Enthält die Mischung neben (Meth)acrylsäure und/oder (Meth)acrylsäureester mit C₁-bis C₈-Alkyl und einer oder mehrerer Verbindungen der allgemeinen Formel (II) noch andere Komponenten, beträgt die Gesamtmenge an (Meth)acrylsäure und (Meth)acrylsäureester mit C₁- bis C₈-Alkyl in der erfindungsgemäßen Mischung bevorzugt mindestens 5 Gew.-% bezogen auf das Gesamtgewicht der Mischung, weiter bevorzugt mindestens 20 Gew.-%, weiter bevorzugt mindestens 30 Gew.-%, weiter bevorzugt mindestens 40 Gew.-%, weiter bevorzugt mindestens 50 Gew.-%, weiter bevorzugt mindestens 60 Gew.-%, weiter bevorzugt mindestens 70 Gew.-%, weiter bevorzugt mindestens 80 Gew.-% , weiter bevorzugt mindestens 90 Gew.-% und besonders bevorzugt mindestens 95 Gew.-%.

Andere Komponenten können beispielsweise Lösungsmittel wie organische Lösungsmittel, Wasser oder Lösungsmittelgemische, eingelöste Gase wie Luft, Nebenkomponenten, Costabilisatoren oder beliebige Mischungen von mehreren dieser Komponenten sein.

Bei Nebenkomponenten handelt es sich beispielsweise um Nebenprodukte, die bei der Herstellung von (Meth)acrylsäure und/oder (Meth)acrylsäureester mit C₁- bis C₈-Alkyl und/oder im Rahmen ihrer Aufreinigung entstanden sind und während der Aufreinigung nicht vollständig abgetrennt wurden. Bei Nebenkomponenten kann es sich auch um unterschiedliche Verschmutzungen handeln, die während Herstellung, Aufreinigung und/oder Lagerung der (Meth)acrylsäure und/oder (Meth)acrylsäureester mit C₁- bis C₈-Alkyl eingetragen wurden. Nebenkomponenten können auf gleichem Wege über die Verbindungen der allgemeinen Formel (II) in die Mischung eingetragen werden.

(Meth)acrylsäure kann abhängig von der Herstellung und Aufreinigung eine oder mehrere Nebenkomponenten enthalten. Nebenkomponenten, die enthalten sein können, sind beispielsweise Wasser, Di(meth)acrylsäure, Essigsäure, Propionsäure, einen oder mehrerer Aldehyde wie Acetaldehyd, Acrolein, Propionaldehyd, Furfural-2, Furfural-3, Cumaron oder Benzaldehyd, Protoanemonin, Maleinsäure, Maleinsäureanhydrid, Allylacrylat oder eine beliebige Mischung aus zwei oder mehr der zuvor genannten Nebenkomponenten. Der Gehalt einer Nebenkomponente liegt in der Regel im Bereich von der erfassbaren Nachweisgrenze bis 20000 Gew.-ppm bezogen auf die Menge an (Meth)acrylsäure.

(Meth)acrylsäureester mit C₁- bis C₈-Alkyl können abhängig von ihrer Herstellung und Aufreinigung eine oder mehrere Nebenkomponenten enthalten. Nebenkomponenten, die enthalten sein können, sind beispielsweise Wasser, (Meth)acrylsäure, Alkohole der jeweiligen (Meth)acrylsäureester, Propionsäureester mit den Alkoholen der jeweiligen (Meth)acrylsäureestern, Acetat-Ester mit den Alkoholen der jeweiligen (Meth)acrylsäureestern, Ether der jeweiligen (Meth)acrylsäureester-Alkohole oder eine beliebige Mischung aus zwei oder mehr der zuvor genannten Nebenkomponenten. Der Gehalt einer Nebenkomponente liegt in der Regel im Bereich von der erfassbaren Nachweisegrenze bis 20000 Gew.-ppm bezogen auf die Menge an (Meth)acrylsäureester.

Die erfindungsgemäße Mischung wird bevorzugt unter einer sauerstoffhaltigen Atomsphäre gelagert und/oder transportiert. Die sauerstoffhaltige Atomsphäre weist bevorzugt einen Sauerstoffgehalt von 5 bis 30 vol.% Sauerstoff, insbesondere einen Sauerstoffgehalt von 5 bis 21 vol.%, auf.

Um eine Sauerstoffsättigung der erfindungsgemäßen Mischung schnellstmöglich zu erreichen und/oder eine kontinuierliche Sauerstoffsättigung sicher zu stellen, kann es bevorzugt sein, dass ein sauerstoffhaltiges Gas in die Mischung eingebracht wird. Das sauerstoffhaltige Gas kann beispielsweise über eine oder mehrere Düsen, deren Öffnungen in die Mischung hineinragen, in die Mischung eingebracht werden.

Bei einem sauerstoffhaltigen Gas handelt es sich beispielsweise um Luft, Stickstoff-Luft-Gemische oder von Luft unterschiedliche Sauerstoff-Stickstoffgemische mit einem Sauerstoffgehalt von 5 bis 30 Vol. Prozent. Neben Sauerstoff und Stickstoff können Sauerstoff-Stickstoffgemische auch andere Gase wie Kohlenstoffdioxid, Kohlenstoffmonoxid, Wasserstoff, Methan, ein oder mehrere Edelgase oder eine Mischung aus zwei oder mehr der zuvor genannten Gase enthalten.

Die Mischung kann dadurch hergestellt werden, dass man eine oder mehrere Verbindungen der allgemeinen Formel (II) mit (Meth)acrylsäure und/oder (Meth)acrylsäureester mit C₁- bis C₈-Alkyl vermischt. Es kann dazu zweckdienlich sein, dass ein Konzentrat von Verbindungen der allgemeinen Formel (II) in (Meth)acrylsäure, (Meth)acrylsäureester mit C₁- bis C₈-Alkyl und/oder in einem geeigneten Lösungsmittel hergestellt wird und das so erhaltene Konzentrat dann mit (Meth)acrylsäure und/oder (Meth)acrylsäureester mit C₁- bis C₈-Alkyl verdünnt wird. Bei der Wahl eins geeigneten Lösungsmittels, das auch ein Lösungsmittelgemisch sein kann, orientiert sich der Fachmann anhand allgemeiner, zweckdienlicher Überlegungen, wie Löslichkeit, Mischbarkeit und potentiell auftretenden Nebeneffekten. Dem Konzentrat können (Meth)acrylsäure und/oder (Meth)acrylsäureester mit C₁- bis C₈-Alkyl zugeführt werden, oder das Konzentrat kann (Meth)acrylsäure und/oder (Meth)acrylsäureester mit C₁- bis C₈-Alkyl zugeführt werden.

Die Mischung enthält bevorzugt einen Costabilisator. Ein Costabilisator kann als chemische Einzelverbindungen oder als Gemisch von Verbindungen in der erfindungsgemäßen Mischung enthalten sein. Ein Costabilisator trägt dazu bei, die radikalische Polymerisation von (Meth)acrylsäure und/oder (Meth)acrylsäureester mit C₁- bis C₈-Alkyl zu unterbinden, beziehungsweise zu verlangsamen. Der Costabilisator kann dabei synergistisch mit den in der Mischung enthaltenden Verbindungen der allgemeinen Formel (II) zusammenwirken, um die radikalische Polymerisation von (Meth)acrylsäure und/oder (Meth)acrylsäureester mit C₁- bis C₈-Alkyl zu unterbinden, beziehungsweise zu verlangsamen. Der Costabilisator kann der Mischung vor und/oder währender der Lagerung und/oder Transport zugegeben werden. Der Costabilisator kann dabei gemeinsam oder separat von den Verbindungen der allgemeinen Formel (II) der (Meth)acrylsäure und/oder (Meth)acrylsäureester mit C₁- bis C₈-Alkyl zugegeben werden. Der Costabilisator kann aber auch bereits bei der Herstellung und/oder Aufreinigung der (Meth)acrylsäure und/oder den (Meth)acrylsäureester mit C₁- bis C₈-Alkyl zugegeben worden sein, wobei Verbindungen der allgemeinen Formel (II) dann anschließend der vorliegenden Mischung zugegeben werden.

Es ist bevorzugt, dass die Gesamtmenge an Costabilisator in den erfindungsgemäßen Mischungen 0,1 bis 5000 Gew.-ppm bezogen auf die Gesamtmenge an (Meth)acrylsäure und (Meth)acrylsäureester mit C₁- bis C₈-Alkyl in der Mischung beträgt. Es ist weiter bevorzugt, dass die Gesamtmenge an Costabilisator 1 bis 4000 Gew.-ppm, insbesondere 5 bis 2500 Gew.-ppm und besonders bevorzugt 50 bis 750 Gew.-ppm jeweils bezogen auf die Gesamtmenge an (Meth)acrylsäure und (Meth)acrylsäureester mit C₁- bis C₈-Alkyl in der Mischung beträgt.

Die Menge an Costabilisator kann durch Zugabe oder Entfernen angepasst werden. Methoden zur Entfernung des Costabilisators sind dem Fachmann bekannt (siehe beispielsweise US 6,046,357). Geeignete Methoden umfassen beispielsweise Destillation, Kristallisation, Ionenaustausch, Extraktion, umgekehrte Osmose und/oder Membranverfahren. Bei der Wahl der geeigneten Methode oder Kombination von Methoden orientiert sich der Fachmann an seinem allgemeinen Fachwissen. Werden Verbindungen der allgemeinen Formel (II) mit abgetrennt, sind diese ggf. wieder zu ergänzen. Gegebenenfalls kann es auch notwendig sein, den Gehalt an Verbindungen der allgemeinen Formel (II) zu erhöhen, um die gewünschte Polymerisationsstabilisierung zu erzielen, wenn der Anteil an Costabilisator in der Mischung reduziert bzw. abgetrennt wird.

Bei dem Costabilisator handelt es sich beispielsweise um Metallsalze aus der Gruppe von Kupfer-, Mangan- und Cer-Salzen, Phenothiazine, phenolische Verbindungen, N-Oxyle, Phenylendiamine, Nitrosoverbindungen, Harnstoffe, Thioharnstoffe, oder um Mischungen aus zwei oder mehr der zuvor genannten Substanzklassen. Bevorzugt handelt es sich bei dem Costabilisator um Phenothiazine, phenolische Verbindungen, N-Oxyle oder um Mischungen aus zwei oder mehr der zuvor genannten Substanzklassen.

Bei Kupfersalzen handelt es sich um Kupfer (I) und/oder Kupfer (II) Salze, die eine ausreichende Löslichkeit in den polymerisationsfähigen Verbindungen und in der erfindungsgemäßen Mischung aufweisen. Geeignete Kupfersalze sind beispielsweise Kupfer(II)phenolat, Kupfer(II)acetylacetonat, Kupfer(II)gluconat, Kupfer(II)tartrat, Kupfer(II)acetat, Kupfer(II)formiat, Kupfer(II)nitrat, Kupfer(II)hydroxid, Kupfer(Il)sulfat, Kupfer(II)carbonat, Kupfer(II)naphthenat, Kupfer(II)acrylat, Kupfer(II)halogenide wie Kupfer(II)chlorid, Kupfer(II)salicylat, Kupfer(II)sulfonat, Kupfer(II)propionat, Kupfer(II)octanoat, Kupfer(I)chlorid, Kupfer(I)iodid, Kupfer(I)bromid, Kupfer(I)cyanid, Kupfer(I)acetat, Kupfer(I)oxid, Kupfer(I)sulfat, komplexe Kupfersalze wie Cu(NH₃)₄⁺ mit entsprechenden Gegenionen, ligandenstabilisierte Kupferkomplexe wie sie in der DE 10 2012 223 695 A1 offenbart sind, oder beliebige Mischungen aus zwei oder mehr der zuvor genannten Kupfersalze. Die entsprechenden Kupfersalze können auch in Form ihrer Hydrate eingesetzt werden.

Bei Mangansalzen handelt es sich bevorzugt um Mangan(II)salze, die eine ausreichende Löslichkeit in den polymerisationsfähigen Verbindungen und in der erfindungsgemäßen Mischung aufweisen. Geeignete Mangansalze sind beispielsweise Mangan(II)phenolat, Mangan(II)acetylacetonat, Mangan(II)gluconat, Mangan(II)tartrat, Mangan(II)acetat, Mangan(II)formiat, Mangan(II)nitrat, Mangan(II)hydroxid, Mangan(II)sulfat, Mangan(II)carbonat, Mangan(II)naphthenat, Mangan(II)acrylat, Mangan(II)halogenide wie Mangan(II)chlorid, Mangan(II)salicylat, Mangan(II)sulfonat, Mangan (II)propionat, Mangan(II)octanoat, ligandenstabilisierte Mangankomplexe wie sie in der DE 10 2012 223 695 A1 offenbart sind, oder beliebige Mischungen aus zwei oder mehr der zuvor genannten Mangansalze. Die entsprechenden Mangansalze können auch in Form ihrer Hydrate eingesetzt werden.

Bei Cersalzen handelt es sich bevorzugt um Cer(III)salze, die eine ausreichende Löslichkeit in den polymerisationsfähigen Verbindungen und in der erfindungsgemäßen Mischung aufweisen. Geeignete Cersalze sind beispielsweise Cer(III)phenolat, Cer(III)acetylacetonat, Cer(III)gluconat, Cer(III)tartrat, Cer(III)acetat, Cer(III)formiat, Cer(III)nitrat, Cer(III)hydroxid, Cer(III)sulfat, Cer(III)carbonat, Cer(III)naphthenat, Cer(III)acrylat, CerIII)halogenide wie Cer(III)chlorid, Cer(III)salicylat, Cer(III)sulfonat, Cer(III)propionat, Cer(III)octanoat, ligandenstabilisierte Cerkomplexe wie sie in der DE 10 2012 223 695 A1 offenbart sind, oder beliebige Mischungen aus zwei oder mehr der zuvor genannten Cersalze. Die entsprechenden Cersalze können auch in Form ihrer Hydrate eingesetzt werden.

Phenothiazine sind beispielsweise Bis-(α-methylbenzyl)phenothiazin, 3,7-Dioctylphenothiazin, Bis-(α-dimethylbenzyl)phenothiazin oder eine beliebige Mischung von zwei oder mehr der zuvor genannten Verbindungen.

Phenolische Verbindungen sind beispielsweise Hydrochinon, Hydrochinonmonomethylether, wie para-Methoxyphenol (MEHQ), Pyrogallol, Catechol, Resorcin, Phenol, Kresol, 2,4-Dimethyl-6-tert-butylphenol, 2,6-Di-tert-butyl-para-kresol oder eine beliebige Mischung von zwei oder mehr der zuvor genannten Verbindungen.

N-Oxyle sind beispielsweise Di-tert-butylnitroxid, 2,2,6,6-Tetramethyl-4-hydroxy-piperidyl-1-oxyl, 2,2,6,6-Tetramethylpiperidyl-1-oxyl, 2,2,6,6-Tetramethylpiperidinoxyl, 4-Hydroxy-2,2,6,6-tetramethylpiperidinoxyl, 4,4',4"-Tris-1-(2,2,6,6-tetramethylpiperidinoxyl)phosphite oder eine beliebige Mischung von zwei oder mehr der zuvor genannten Verbindungen.

Ein weiterer Gegenstand der vorliegenden Anmeldung ist die Verwendung einer oder mehrerer Verbindungen der allgemeinen Formel (II) als Lager- und/oder Transportstabilisator von (Meth)acrylsäure und/oder (Meth)acrylsäureester mit C₁- bis C₈-Alkyl. Auf die bereits beschriebenen bevorzugten Ausführungsformen wird in vollem Umfang Bezug genommen. Damit sind besonders bevorzugte, ganz besonders bevorzugte oder durch ähnliche Formulierungen als bevorzugt gekennzeichnete Ausführungsformen miterfasst.

Weiterhin ist ein Verfahren zur Stabilisierung von (Meth)acrylsäure und/oder (Meth)acrylsäureester mit C₁- bis C₈-Alkyl bei Lagerung und/oder Transport unter Verwendung einer oder mehrerer Verbindungen der allgemeinen Formel (II) Gegenstand der vorliegenden Anmeldung. Auf die bereits beschriebenen bevorzugten Ausführungsformen wird in vollem Umfang Bezug genommen. Damit sind besonders bevorzugte, ganz besonders bevorzugte oder durch ähnliche Formulierungen als bevorzugt gekennzeichnete Ausführungsformen miterfasst.

Das erfindungsgemäße Verfahren umfasst das Bereitstellen einer Mischung, die eine oder mehrere Verbindungen der allgemeinen Formel (II) und (Meth)acrylsäure und/oder (Meth)acrylsäureester mit C₁- bis C₈-Alkyl enthält, wobei die Mischung mit Sauerstoff zu 50 bis 100%, bevorzugt zu 90 bis 100 % und besonders bevorzugt zu 95 bis 100% gesättigt ist. Der Grad der Sauerstoffsättigung bezieht auf den Grad der Sauerstoffsättigung der im Gleichgewicht erreicht wird, wenn die Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.

Ein weiterer Gegenstand der vorliegenden Erfindung ist ein Verfahren zur Polymerisation von (Meth)acrylsäure und/oder (Meth)acrylsäureester mit C₁- bis C₈-Alkyl in Gegenwart einer oder mehrerer Verbindungen der allgemeinen Formel (II), wobei die Reaktionsmischung zu 0 bis 50 %, bevorzugt zu 0 bis 10 %, besonders bevorzugt zu 0 bis 5 %, weiter bevorzugt zu 0 bis 1 %, ganz besonders bevorzugt zu 0 bis 0,1 % mit Sauerstoff gesättigt ist. Der Grad der Sauerstoffsättigung bezieht auf den Grad der Sauerstoffsättigung der erreicht wird, wenn die Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.

Ist die Sauerstoffsättigung der Reaktionsmischung zu hoch, kann die Sauerstoffsättigung beispielsweise durch Einleiten von Stickstoff in die Reaktionsmischung und/oder in die Atmosphäre oberhalb der Reaktionsmischung reduziert werden. Der eingeleitete Stickstoff sollte dabei weitgehend frei von Sauerstoff sein, beispielsweise einen Sauerstoffgehalt von weniger als 1 vol.%, bevorzugt weniger als 0,1 vol.% aufweisen.

Neben (Meth)acrylsäure und/oder (Meth)acrylsäureester mit C₁- bis C₈-Alkyl kann die Reaktionsmischung noch weitere polymerisationsfähige Verbindungen enthalten.

Auf die bereits beschriebenen bevorzugten Ausführungsformen wird in vollem Umfang Bezug genommen. Damit sind besonders bevorzugte, insbesonders bevorzugte oder durch ähnliche Formulierungen als bevorzugt gekennzeichnete Ausführungsformen miterfasst.

Es ist bevorzugt, dass die Polymerisation bei einer Temperatur von 50 bis 150°C und weiter bevorzugt bei einer Temperatur von 70 bis 120°C durchgeführt wird.

Es ist bevorzugt, dass die Polymerisation unter einer Stickstoffatomsphäre durchgeführt wird. Eine Stickstoffatomsphäre weist einen Stickstoffgehalt von 85 bis 100 vol.%, bevorzugt 90 bis 100 vol.% und besonders bevorzugt 99 bis 100 vol.% auf. Der Sauerstoffgehalt der Stickstoffatomsphäre ist unter 5 vol.%.

Es ist bevorzugt, dass die Polymerisation in Gegenwart eines Polymerisationsstarters durchgeführt wird. Als Polymerisationsstarter eignen sich die dem Fachmann bekannten Polymerisationsstarter wie sie beispielsweise in The Chemistry of Radical Polymerisation, 2nd Edition, 2005, Seite 49 bis 166 beschrieben sind. Der Polymerisationsstarter wird in wirksamen Mengen eingesetzt.

Durch das erfindungsgemäße Verfahren ist es möglich, (Meth)acrylsäure und/oder (Meth)acrylsäureester mit C₁- bis C₈-Alkyl, die mit einer oder mehrerer Verbindungen der allgemeinen Formel (II) lager- und/oder transportstabilisiert sind zu polymerisieren, ohne die Notwendigkeit die Verbindungen der allgemeinen Formel (II) zuvor von der zu polymerisierenden Reaktionsmischung abzutrennen. Die Reaktionsmischung kann somit stabilisiert im Reaktor zur Polymerisation vorgelegt werden. Durch Absenken der Sauerstoffsättigung in der Reaktionsmischung, bevorzugt durch Einleiten von Stickstoff in den Reaktorraum oberhalb der Reaktionsmischung und/oder durch Einleiten von Stickstoff in die Reaktionsmischung kann die Stabilisierungswirkung der Verbindungen der allgemeinen Formel (II) derart reduziert werden, dass die Reaktionsmischung polymerisiert werden kann. Die Zugabe des Polymerisationsstarters kann vor, während oder nach dem Absenken der Sauerstoffsättigung erfolgen. Aus Sicherheitsgründen ist es bevorzugt, wenn der Polymerisationsstarter vor dem Absenken der Sauerstoffsättigung zugegeben wird.

Der Gegenstand der vorliegenden Erfindung umfasst besonders die nachfolgenden Ausführungsformen.
1. Mischung enthaltend eine oder mehrere Verbindungen der allgemeinen Formel (II) 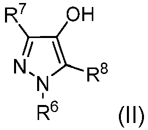 wobei
   R⁶ H oder C₁- bis C₆-Alkyl ist, wobei C₁- bis C₆-Alkyl gerade oder verzweigte C₁-bis C₆-Alkylgruppen oder cyclische C₃- bis C₆-Alkylgruppen umfasst,
   R⁷ H, C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst, oder Alkanoyl mit C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst, und
   R⁸ H oder C₁- bis C₆-Alkyl ist, wobei C₁- bis C₆-Alkyl gerade oder verzweigte C₁-bis C₆-Alkylgruppen oder cyclische C₃- bis C₆-Alkylgruppen umfasst,
   und (Meth)acrylsäure und/oder (Meth)acrylsäureester mit C₁- bis C₈-Alkyl, wobei C₁- bis C₈-Alkyl gerade oder verzweigte C₁- bis C₈-Alkylgruppen oder cyclische C₃- bis C₈-Alkylgruppen umfasst.
2. Mischung nach einer der Ausführungsform 1, wobei es sich bei (Meth)acrylsäureester mit C₁- bis C₈-Alkyl um Methyl(meth)acrylat, Ethyl(meth)acrylat, n-Butyl(meth)acrylat, 2-Ethylhexyl(meth)acrylat oder um eine Mischung aus zwei oder mehr der zuvor genannten (Meth)acrylsäureester handelt.
3. Mischung nach Ausführungsform 1 oder 2, wobei die Gesamtmenge der Verbindungen der allgemeinen Formel (II) in der Mischung 0,1 bis 1000 Gew.-ppm beträgt, bevorzugt 1 bis 900 Gew.-ppm, besonders bevorzugt 10 bis 800 Gew.-ppm und insbesondere 10 bis 500 Gew.-ppm beträgt, jeweils bezogen auf das Gesamtgewicht der in der Mischung enthaltenen Menge an (Meth)acrylsäure und (Meth)acrylsäureester mit C₁- bis C₈-Alkyl.
4. Mischung nach einer der Ausführungsformen 1 bis 3, wobei die Gesamtmenge an (Meth)acrylsäure und (Meth)acrylsäureester mit C₁- bis C₈-Alkyl in der Mischung mindestens 5 Gew.-%, weiter bevorzugt mindestens 20 Gew.-%, weiter bevorzugt mindestens 30 Gew.-%, weiter bevorzugt mindestens 40 Gew.-%, weiter bevorzugt mindestens 50 Gew.-%, weiter bevorzugt mindestens 60 Gew.-%, weiter bevorzugt mindestens 70 Gew.-%, weiter bevorzugt mindestens 80 Gew.-% , weiter bevorzugt mindestens 90 Gew.-% und besonders bevorzugt mindestens 95 Gew.-%, jeweils bezogen auf das Gesamtgewicht der Mischung beträgt.
5. Mischung nach einer der Ausführungsformen 1 oder 3 bis 4, wobei die Mischung (Meth)acrylsäure enthält und wobei die Mischung eine oder mehrere der nachfolgenden Komponenten enthält:
   a. Wasser 20 bis 15000 Gew.-ppm bezogen auf das Gesamtgewicht der in der Mischung enthaltenen (Meth)acrylsäure,
   b. Di(meth)acrylsäure 100 bis 20000 Gew.-ppm bezogen auf das Gesamtgewicht der in der Mischung enthaltenen (Meth)acrylsäure,
   c. Essigsäure 100 bis 2000 Gew.-ppm bezogen auf das Gesamtgewicht der in der Mischung enthaltenen (Meth)acrylsäure,
   d. Propionsäure 100 bis 2000 Gew.-ppm bezogen auf das Gesamtgewicht der n der Mischung enthaltenen (Meth)acrylsäure,
   e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew. ppm bezogen auf das Gesamtgewicht der in der Mischung enthaltenen (Meth)acrylsäure beträgt,
   f. Protoanemonin 0 bis 2 Gew.-ppm bezogen auf das Gesamtgewicht der in der Mischung enthaltenen (Meth)acrylsäure,
   g. Maleinsäure 0 bis 1 Gew.-ppm bezogen auf das Gesamtgewicht der in der Mischung enthaltenen (Meth)acrylsäure,
   h. Maleinsäureanhydrid 0 bis 1 Gew.-ppm bezogen auf das Gesamtgewicht der in der Mischung enthaltenen (Meth)acrylsäure,
   i. Allylacrylat 0 bis 30 Gew.-ppm, bevorzugt 0 bis 1 Gew.-ppm bezogen auf das Gesamtgewicht der in der Mischung enthaltenen (Meth)acrylsäure.
6. Mischung nach einer der Ausführungsformen 2 bis 4, wobei die Mischung einen oder mehrere (Meth)acrylsäureester gemäß der Ausführungsform 2 enthält und wobei die Mischung eine oder mehrere der nachfolgenden Komponenten enthält:
   a. Wasser 1 bis 2000 Gew.-ppm, bevorzugt 1 bis 500 Gew.-ppm jeweils bezogen auf das Gesamtgewicht der in der Mischung enthaltenen (Meth)acrylsäureester gemäß der Ausführungsform 2,
   b. (Meth)acrylsäure 1 bis 2000 Gew.-ppm, bevorzugt 1 bis 100 Gew.-ppm jeweils bezogen auf das Gesamtgewicht der in der Mischung enthaltenen (Meth)acrylsäureester gemäß der Ausführungsform 2,
   c. Alkohole der jeweiligen (Meth)acrylsäureester 0,01 bis 2000 Gew.-ppm, bevorzugt 0,1 bis 1000 Gew.-ppm jeweils bezogen auf das Gesamtgewicht der in der Mischung enthaltenen (Meth)acrylsäureester gemäß der Ausführungsform 2,
   d. Propionsäureester mit den Alkoholen der jeweiligen (Meth)acrylsäureester 0,1 bis 2000 Gew.-ppm, bevorzugt 10 bis 1000 Gew.-ppm jeweils bezogen auf das Gesamtgewicht der in der Mischung enthaltenen (Meth)acrylsäureester gemäß der Ausführungsform 2,
   e. Essigsäureester mit den Alkoholen der jeweiligen (Meth)acrylsäureester 1 bis 2000 Gew.-ppm, bevorzugt 5 bis 2000 Gew.-ppm jeweils bezogen auf das Gesamtgewicht der in der Mischung enthaltenen (Meth)acrylsäureester gemäß der Ausführungsform 2,
   f. Ether der jeweiligen (Meth)acrylsäureester-Alkohole 0 bis 2000 Gew.-ppm bezogen auf das Gesamtgewicht der in der Mischung enthaltenen (Meth)acrylsäureester gemäß der Ausführungsform 2.
7. Mischung nach einer der Ausführungsformen 1 bis 6, wobei die Mischung unter einer sauerstoffhaltigen Atomsphäre gelagert und/oder transportiert wird.
8. Mischung nach Ausführungsform 7, wobei die sauerstoffhaltige Atomsphäre einen Sauerstoffgehalt von 5 bis 30 vol.% Sauerstoff und bevorzugt einen Sauerstoffgehalt von 5 bis 21 vol.% aufweist.
9. Mischung nach Ausführungsform 7 oder 8, wobei die Mischung für 10 Sekunden oder mehr, bevorzugt für 1 Minute oder mehr, weiter bevorzugt für 10 Minuten oder mehr und besonders bevorzugt für 60 Minuten oder mehr unter einer sauerstoffhalten Atmosphäre gelagert und/oder transportiert wird.
10. Mischung nach einer der Ausführungsformen 1 bis 9, wobei die Mischung einen Costabilisator enthält.
11. Mischung nach Ausführungsform 10, wobei die in der Mischung enthaltene Gesamtmenge an Costabilisator 0,1 bis 5000 Gew.-ppm beträgt, bevorzugt, 1 bis 4000 Gew.-ppm, insbesondere 5 bis 2500 Gew.-ppm und weiter bevorzugt 50 bis 750 Gew.-ppm, bezogen auf die Gesamtgewicht der in der Mischung enthaltenen Menge an (Meth)acrylsäure und (Meth)acrylsäureester mit C₁- bis C₈-Alkyl.
12. Mischung nach einer der Ausführungsformen 10 oder 11, wobei es sich bei dem Costabilisator um Metallsalze aus der Gruppe von Kupfer-, Mangan- und Cer-Salzen, Phenothiazine, phenolische Verbindungen, N-Oxyle, Phenylendiamine, Nitrosoverbindungen, Harnstoffe, Thioharnstoffe, oder um Mischungen aus zwei oder mehr der zuvor genannten Substanzklassen handelt.
13. Verwendung einer oder mehrerer Verbindungen der allgemeinen Formel (II) wie in einer der Ausführungsform 1 definiert zur Lager- und/oder Transportstabilisierung von (Meth)acrylsäure und/oder (Meth)acrylsäureester mit C₁- bis C₈-Alkyl.
14. Verwendung nach Ausführungsform 13, wobei es sich bei (Meth)acrylsäureester mit C₁- bis C₈-Alkyl um Methyl(meth)acrylat, Ethyl(meth)acrylat, n-Butyl(meth)acrylat, 2-Ethylhexyl(meth)acrylat oder um eine Mischung aus zwei oder mehr der zuvor genannten (Meth)acrylsäureester handelt.
15. Verwendung nach einer der Ausführungsformen 13 oder 14, wobei die Verbindungen der allgemeinen Formel (II) in einer Gesamtmenge von 0,1 bis 1000 Gew.-ppm, bevorzugt 1 bis 900 Gew.-ppm, besonders bevorzugt 10 bis 800 Gew.-ppm und insbesondere 10 bis 500 Gew.-ppm, bezogen auf das Gesamtgewicht der zu stabilisierenden Menge an (Meth)acrylsäure und (Meth)acrylsäureester mit C₁- bis C₈-Alkyl eingesetzt werden.
16. Verwendung nach einer der Ausführungsformen 13 oder 15, wobei (Meth)acrylsäure stabilisiert wird und wobei die zu stabilisierende (Meth)acrylsäure eine oder mehrere der nachfolgenden Komponenten enthält:
   a. Wasser 20 bis 15000 Gew.-ppm bezogen auf die Menge an (Meth)acrylsäure,
   b. Di(meth)acrylsäure 100 bis 20000 Gew.-ppm bezogen auf die Menge an (Meth)acrylsäure,
   c. Essigsäure 100 bis 2000 Gew.-ppm bezogen auf die Menge an (Meth)acrylsäure,
   d. Propionsäure 100 bis 2000 Gew. -ppm bezogen auf die Menge an (Meth)acrylsäure,
   e. ein oder mehrere Aldehyde wie Acetaldehyd, Acrolein, Propionaldehyd, Furfural-2, Furfural-3, Cumaron oder Benzaldehyd, wobei die Menge pro Aldehyde 0 bis 1 Gew.-ppm bezogen auf die Menge an (Meth)acrylsäure beträgt,
   f. Protoanemonin 0 bis 2 Gew.-ppm bezogen auf die Menge an (Meth)acrylsäure,
   g. Maleinsäure 0 bis 1 Gew.-ppm bezogen auf die Menge an (Meth)acrylsäure,
   h. Maleinsäureanhydrid 0 bis 1 Gew.-ppm bezogen auf die Menge an (Meth)acrylsäure,
   i. Allylacrylat 0 bis 30 Gew.-ppm, bevorzugt 0 bis 1 Gew.-ppm jeweils bezogen auf die Menge an (Meth)acrylsäure.
17. Verwendung nach einer der Ausführungsformen 14 oder 15, wobei ein oder mehrere (Meth)acrylsäureester gemäß der Ausführungsform 14 stabilisiert werden und wobei die zu stabilisierenden (Meth)acrylsäureester eine oder mehrere der nachfolgenden Komponenten enthalten:
   a. Wasser 1 bis 2000 Gew.-ppm, bevorzugt 1 bis 500 Gew.-ppm jeweils bezogen auf die Menge an (Meth)acrylsäureester gemäß der Ausführungsform 14,
   b. (Meth)acrylsäure 1 bis 2000 Gew.-ppm, bevorzugt 1 bis 100 Gew.-ppm jeweils bezogen auf die Menge an (Meth)acrylsäureester gemäß der Ausführungsform 14,
   c. Alkohole der jeweiligen (Meth)acrylsäureester 0,01 bis 2000 Gew.-ppm, bevorzugt 0,1 bis 1000 Gew.-ppm jeweils bezogen auf die Menge an (Meth)acrylsäureester gemäß der Ausführungsform 14,
   d. Propionsäureester mit den Alkoholen der jeweiligen (Meth)acrylsäureester 0,1 bis 2000 Gew.-ppm, bevorzugt 10 bis 1000 Gew.-ppm jeweils bezogen auf die Menge an (Meth)acrylsäureester gemäß der Ausführungsform 14,
   e. Essigsäureester mit den Alkoholen der jeweiligen (Meth)acrylsäureester 1 bis 2000 Gew.-ppm, bevorzugt 5 bis 2000 Gew.-ppm jeweils bezogen auf die Menge an (Meth)acrylsäureester gemäß der Ausführungsform 14,
   f. Ether der jeweiligen (Meth)acrylsäureester-Alkohole 0 bis 2000 Gew.-ppm bezogen auf die Menge an (Meth)acrylsäureester gemäß der Ausführungsform 14.
18. Verfahren zur Polymerisation von (Meth)acrylsäure und/oder (Meth)acrylsäureester mit C₁- bis C₈-Alkyl in Gegenwart einer oder mehrerer Verbindungen der allgemeinen Formel (II) wie in einer der Ausführungsform 1 definiert, wobei die Reaktionsmischung zu 0 bis 50 %, bevorzugt zu 0 bis 10 %, weiter bevorzugt zu 0 bis 5 %, besonders bevorzugt 0 bis 1% und ins besonders bevorzugt zu 0 bis 0,1 % mit Sauerstoff gesättigt ist, wobei der Grad der Sauerstoffsättigung sich auf den Grad der Sauerstoffsättigung bezieht der im Gleichgewicht erreicht wird, wenn die vorliegende Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.
19. Verfahren nach Ausführungsform 18, wobei die Gesamtmenge der Verbindungen der allgemeinen Formel (II) in der Reaktionsmischung 0,1 bis 1000 Gew.-ppm, bevorzugt 1 bis 900 Gew.-ppm, besonders bevorzugt 10 bis 800 Gew.-ppm und insbesondere 10 bis 500 Gew.-ppm beträgt, jeweils bezogen auf das Gesamtgewicht der in der Reaktionsmischungen enthalten Menge an (Meth)acrylsäure und (Meth)acrylsäureester mit C₁- bis C₈-Alkyl.
20. Verfahren nach einer der Ausführungsformen 18 oder 19, wobei die Polymerisation bei einer Temperatur von 50 bis 150°C und bevorzugt 70 bis 120°C durchgeführt wird.
21. Verfahren nach einer der Ausführungsformen 18 bis 20, wobei die Polymerisation unter einer Stickstoffatomsphäre mit einem Stickstoffgehalt von 85 bis 100 vol.% und einem Sauerstoffgehalt von weniger als 5 vol.%, bevorzugt mit einem Stickstoffgehalt von 90 bis 100 vol.% und besonders bevorzugt mit einem Stickstoffgehalt von 99 bis 100 vol.% durchgeführt wird.
22. Verfahren nach einer der Ausführungsformen 18 bis 21, wobei es sich bei (Meth)acrylsäureester mit C₁- bis C₈-Alkyl um Methyl(meth)acrylat, Ethyl(meth)acrylat, n-Butyl(meth)acrylat, 2-Ethylhexyl(meth)acrylat oder um eine Mischung aus zwei oder mehr der zuvor genannten (Meth)acrylsäureester handelt.
23. Verfahren nach einer der Ausführungsformen 18 bis 21, wobei die zu polymerisierende (Meth)acrylsäure eine oder mehrere der nachfolgenden Komponenten enthält:
   a. Wasser 20 bis 15000 Gew.-ppm bezogen auf die Menge an (Meth)acrylsäure,
   b. Di(meth)acrylsäure 100 bis 20000 Gew.-ppm bezogen auf die Menge an (Meth)acrylsäure,
   c. Essigsäure 100 bis 2000 Gew.-ppm bezogen auf die Menge an (Meth)acrylsäure,
   d. Propionsäure 100 bis 2000 Gew.-ppm bezogen auf die Menge an (Meth)acrylsäure,
   e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew.-ppm bezogen auf die Menge an (Meth)acrylsäure beträgt,
   f. Protoanemonin 0 bis 2 Gew.-ppm bezogen auf die Menge an (Meth)acrylsäure,
   g. Maleinsäure 0 bis 1 Gew.-ppm bezogen auf die Menge an (Meth)acrylsäure,
   h. Maleinsäureanhydrid 0 bis 1 Gew.-ppm bezogen auf die Menge an (Meth)acrylsäure,
   i. Allylacrylat 0 bis 30 Gew.-ppm, bevorzugt 0 bis 1 Gew.-ppm bezogen auf die Menge an (Meth)acrylsäure.
24. Verfahren nach Ausführungsform 22, wobei die zu polymerisierenden (Meth)acrylsäureester eine oder mehrere der nachfolgenden Komponenten enthalten:
   a. Wasser 1 bis 2000 Gew.-ppm, bevorzugt 1 bis 500 Gew.-ppm jeweils bezogen auf die Menge an (Meth)acrylsäureester gemäß Ausführungsform 22,
   b. (Meth)acrylsäure 1 bis 2000 Gew.-ppm, bevorzugt 1 bis 100 Gew.-ppm, jeweils bezogen auf die Menge an (Meth)acrylsäureester gemäß Ausführungsform 22,
   c. Alkohole der jeweiligen (Meth)acrylsäureester 0,01 bis 2000 Gew.-ppm, bevorzugt 0,1 bis 1000 Gew.-ppm jeweils bezogen auf die Menge an (Meth)acrylsäureester gemäß Ausführungsform 22,
   d. Propionsäureester mit den Alkoholen der jeweiligen (Meth)acrylsäureester 0,1 bis 2000 Gew.-ppm, bevorzugt 10 bis 1000 Gew.-ppm jeweils bezogen auf die Menge an (Meth)acrylsäureester gemäß Ausführungsform 22,
   e. Essigsäureester mit den Alkoholen der jeweiligen (Meth)acrylsäureester 1 bis 2000 Gew.-ppm, bevorzugt 5 bis 2000 Gew.-ppm jeweils bezogen auf die Menge an (Meth)acrylsäureester gemäß Ausführungsform 22,
   f. Ether der jeweiligen (Meth)acrylsäureester-Alkohole 0 bis 2000 Gew.-ppm bezogen auf die Menge an (Meth)acrylsäureester gemäß Ausführungsform 24.
25. Verfahren zur Stabilisierung von (Meth)acrylsäure und/oder (Meth)acrylsäureester mit C₁- bis C₈-Alkyl unter Verwendung einer oder mehrerer Verbindungen der allgemeinen Formel (II) wie in einer der Ausführungsform 1 definiert.
26. Verfahren nach Ausführungsform 25, wobei (Meth)acrylsäure und/oder (Meth)acrylsäureester mit C₁- bis C₈-Alkyl bei Lagerung und/oder Transport stabilisiert werden.
27. Verfahren nach einer der Ausführungsformen 25 oder 26, wobei es sich bei (Meth)acrylsäureester mit C₁- bis C₈-Alkyl um Methyl(meth)acrylat, Ethyl(meth)acrylat, n-Butyl(meth)acrylat, 2-Ethylhexyl(meth)acrylat oder um eine Mischung aus zwei oder mehr der zuvor genannten (Meth)acrylsäureester handelt.
28. Verfahren nach einer der Ausführungsformen 25 oder 26, wobei (Meth)acrylsäure eine oder mehrere der nachfolgenden Komponenten enthält:
   a. Wasser 20 bis 15000 Gew.-ppm bezogen auf die Menge an (Meth)acrylsäure,
   b. Di(meth)acrylsäure 100 bis 20000 Gew.-ppm bezogen auf die Menge an (Meth)acrylsäure,
   c. Essigsäure 100 bis 2000 Gew.-ppm bezogen auf die Menge an (Meth)acrylsäure,
   d. Propionsäure 100 bis 2000 Gew.-ppm bezogen auf die Menge an (Meth)acrylsäure,
   e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew.-ppm bezogen auf die Menge an (Meth)acrylsäure beträgt,
   f. Protoanemonin 0 bis 2 Gew.-ppm bezogen auf die Menge an (Meth)acrylsäure,
   g. Maleinsäure 0 bis 1 Gew.-ppm bezogen auf die Menge an (Meth)acrylsäure,
   h. Maleinsäureanhydrid 0 bis 1 Gew.-ppm bezogen auf die Menge an (Meth)acrylsäure,
   i. Allylacrylat 0 bis 30 Gew.-ppm, bevorzugt 0 bis 1 Gew.-ppm bezogen auf die Menge an (Meth)acrylsäure.
29. Verfahren nach Ausführungsform 27, wobei die (Meth)acrylsäureester eine oder mehrere der nachfolgenden Komponenten enthalten:
   a. Wasser 1 bis 2000 Gew.-ppm, bevorzugt 1 bis 500 Gew.-ppm jeweils bezogen auf die Menge an (Meth)acrylsäureester gemäß Ausführungsform 27,
   b. (Meth)acrylsäure 1 bis 2000 Gew.-ppm, bevorzugt 1 bis 100 Gew.-ppm jeweils bezogen auf die Menge an (Meth)acrylsäureester gemäß Ausführungsform 27,
   c. Alkohole der jeweiligen (Meth)acrylsäureester 0,01 bis 2000 Gew.-ppm, bevorzugt 0,1 bis 1000 Gew.-ppm jeweils bezogen auf die Menge an (Meth)acrylsäureester gemäß Ausführungsform 27,
   d. Propionsäureester mit den Alkoholen der jeweiligen (Meth)acrylsäureester 0,1 bis 2000 Gew.-ppm, bevorzugt 10 bis 1000 Gew.-ppm jeweils bezogen auf die Menge an (Meth)acrylsäureester gemäß Ausführungsform 27,
   e. Essigsäureester mit den Alkoholen der jeweiligen (Meth)acrylsäureester 1 bis 2000 Gew.-ppm, bevorzugt 5 bis 2000 Gew.-ppm jeweils bezogen auf die Menge an (Meth)acrylsäureester gemäß Ausführungsform 27,
   f. Ether der jeweiligen (Meth)acrylsäureester-Alkohole 0 bis 2000 Gew.-ppm bezogen auf die Menge an (Meth)acrylsäureester gemäß Ausführungsform 27.
30. Verfahren nach einer der Ausführungsformen 25 bis 29, wobei das Verfahren das Bereitstellen einer Mischung, die eine oder mehrere Verbindungen der allgemeinen Formel (II) und (Meth)acrylsäure und/oder (Meth)acrylsäureester mit C₁- bis C₈-Alkyl enthält umfasst, wobei die Mischung mit Sauerstoff zu 50 bis 100%, bevorzugt zu 90 bis 100% und besonders bevorzugt zu 95 bis 100 % gesättigt ist, wobei der Grad der Sauerstoffsättigung sich auf den Grad der Sauerstoffsättigung bezieht der im Gleichgewicht erreicht wird, wenn die vorliegende Mischung unter Standardbedingungen unter einer sauerstoffhaltigen Atmosphäre mit einem Sauerstoffanteil von 5 bis 30 vol.% und bevorzugt mit einem Sauerstoffanteil von 5 bis 21 vol.% gelagert und/oder transportiert wird.
31. Verfahren nach Ausführungsform 30, wobei die Gesamtmenge an (Meth)acrylsäure und (Meth)acrylsäureester mit C₁- bis C₈-Alkyl in der Mischung mindestens 5 Gew.-% bezogen auf das Gesamtgewicht der Mischung, weiter bevorzugt mindestens 20 Gew.-%, weiter bevorzugt mindestens 30 Gew.-%, weiter bevorzugt mindestens 40 Gew.-%, weiter bevorzugt mindestens 50 Gew.-%, weiter bevorzugt mindestens 60 Gew.-%, weiter bevorzugt mindestens 70 Gew.-%, weiter bevorzugt mindestens 80 Gew.-% , weiter bevorzugt mindestens 90 Gew.-% und besonders bevorzugt mindestens 95 Gew.-% beträgt.
32. Verfahren nach einer der Ausführungsformen 25 bis 31, wobei die Verbindungen der allgemeinen Formel (II) in einer Gesamtmenge von 0,1 bis 1000 Gew.-ppm, bevorzugt 1 bis 900 Gew.-ppm, besonders bevorzugt 10 bis 800 Gew.-ppm und insbesondere 10 bis 500 Gew.-ppm, jeweils bezogen auf das Gesamtgewicht der in der Mischung enthaltenen (Meth)acrylsäure und (Meth)acrylsäureester mit C₁- bis C₈-Alkyl eingesetzt werden.

### Beispiele:

Die nachfolgenden Beispiele schränken den Gegenstand der vorliegenden Erfindung nicht ein.

| Einsatzstoff | Hersteller | Reinheit |
|---|---|---|
| Acrylsäure | BASF SE | ≥99% |
| Toluol | BASF SE | >99,7 % |
| 2,2'-Azobis(4-methoxy-2,4-dimethylvaleronitril) | FUJIFILM Wako Chemicals Europe GmbH | n.a. |
| 4-Methoxyphenol | Sigma-Aldrich | ≥98% |
| Ethylacrylat | BASF SE | ≥99,7% |

Die in den Versuchen eingesetzte Acrylsäure wurde frisch destilliert, um den darin enthaltenen Stabilisator zu entfernen. Die destillierte Acrylsäure weist eine Reinheit von größer 99,5 Gew. % auf. In der destillierten Acrylsäure enthaltene Nebenkomponenten sind Essigsäure (0,0726 Gew.-%), Diacrylsäure (0,0497 Gew.-%) und Propionsäure (0,0184 Gew. %).

Gewichtsangaben in den Versuchsbeschreibungen, wie Gewichtsprozent (Gew.-%) oder Gew.-ppm, die auf Acrylsäure bezogen sind, beziehen sich auf die Masse der jeweils eingesetzten Acrylsäure incl. der darin enthaltenen Nebenkomponenten.

Versuche zur Polymerisationsinhibierung unter Luft oder Stickstoff:
Für die Vergleichsversuche wurde eine 40 Gew.-ppm 4-Methoxyphenol (MEHQ) enthaltende Acrylsäurelösung hergestellt. Die Angabe Gew.-ppm bezieht sich auf die Masse der eingesetzten Acrylsäure.

Für weitere Vergleichsversuche wurden Mischungen von Acrylsäure mit Verbindungen, die von den Verbindungen der allgemeinen Formel (II) unterschiedlich sind, hergestellt, wobei die Molmenge der eingesetzten Verbindungen der Molmenge an MEHQ in den Vergleichsversuchen entspricht.

Für die erfindungsgemäßen Versuche wurden Mischungen von Acrylsäure mit Verbindungen der allgemeinen Formel (II) hergestellt, wobei die Molmenge an Verbindungen der allgemeinen Formel (II) in den Mischungen der Molmenge an MEHQ in den Vergleichsversuchen entspricht.

Exemplarische Verdünnungsreihe:
Von einer Acrylsäurelösung mit einem Gehalt von 1000 Gew.-ppm MEHQ bezogen auf die Masse der eingesetzten Acrylsäure wurden 2,5 g entnommen und mit Acrylsäure auf einen Gehalt von 500 Gew.-ppm MEHQ bezogen auf die Masse der Acrylsäure verdünnt.

Von der so erhaltenen Acrylsäurelösung wurden 2,5 g entnommen und mit Acrylsäure auf einen Gehalt von 250 Gew.-ppm MEHQ bezogen auf die Masse der Acrylsäure verdünnt.

Von der so erhaltenen Acrylsäurelösung wurden 2,5 g entnommen und mit Acrylsäure auf einen Gehalt von 125 Gew.-ppm MEHQ bezogen auf die Masse der Acrylsäure verdünnt.

Von der so erhaltenen Acrylsäurelösung wurden 2,5 g entnommen und mit Acrylsäure auf einen Gehalt von 80 Gew.-ppm MEHQ bezogen auf die Masse der Acrylsäure verdünnt.

Von der so erhaltenen Acrylsäurelösung wurden 2,5 g entnommen und mit Acrylsäure auf einen Gehalt von 40 Gew.-ppm MEHQ bezogen auf die Masse der Acrylsäure verdünnt.

Als Starterlösung wurde eine frisch angesetzte Toluol-Lösung von 2,2'-Azobis(4-methoxy-2,4-dimethylvaleronitril) verwendet. Der Gehalt an 2,2'-Azobis(4-methoxy-2,4-dimehtylvaleronitril) in der Starterlösung beträgt 6420 Gew.-ppm bezogen auf die Masse an Toluol in der Starterlösung.

### Tests zur Polymerisationsinhibierung unter Luft:

Unter Luft wurden 2.2 g Acrylsäurelösung enthaltend 0,000715 mmol des jeweiligen Polymerisationsstabilisators vorgelegt. Die vorgelegte Acrylsäurelösung wurde mit 0,22 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß.

Das Reaktionsgefäß enthaltend die Mischung wurde anschließend in einem auf 40°C erwärmten Heizblock platziert.

Nachdem die Mischung die Temperatur von 40°C erreicht hat wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 42 Sekunden dauernder Temperaturanstieg um 145°C auf eine maximale Temperatur von 185°C gemessen. Die maximale Temperatur lag nach 9188 Sekunden vor, nachdem die Mischung auf 40°C temperiert war. Zur Berechnung der Effizienz der Polymerisationsstabilisatoren in Bezug auf MEHQ entspricht der Wert von 9188 Sekunden einer Effizienz von 100 %.

Für eine Mischung mit 5-Methyl-2-phenyl-4H-pyrazol-3-on als Polymerisationsstabilisator wurde ein 48 Sekunden dauernder Temperaturanstieg um 125°C auf eine maximale Temperatur von 165°C beobachtet. Die maximale Temperatur lag nach 7874 Sekunden vor, nachdem die Mischung auf 40°C temperiert war.

Im Vergleich zu MEHQ weist 5-Methyl-2-phenyl-4H-pyrazol-3-on als Polymerisationsstabilisator somit eine Effizienz von 86 Prozent auf.

### Tests zur Polymerisationsinhibierung unter Stickstoff:

Unter Luft wurden 2,2 g Acrylsäurelösung enthaltend 0,000715 mmol des jeweiligen Polymerisationsstabilisators vorgelegt. Die vorgelegte Acrylsäurelösung wurde mit 0,22 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß. In das Reaktionsgefäß wurde oberhalb der vorgelegten Mischung Stickstoff unter Druckausgleich eingeleitet. Der Stickstoffstrom beträgt 3 bis 4 l/h. Die Einleitdauer beträgt 20 Sekunden. Der Stickstoff weist eine Reinheit von größer 99,9 % auf.

Das Reaktionsgefäß enthaltend die Mischung wurde in einem Heizblock platziert. Die Mischung wurde anschließend im Heizblock innerhalb von 4 Minuten auf 40°C erwärmt.

Nachdem die Mischung die Temperatur von 40°C erreicht hat, wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 54 Sekunden dauernder Temperaturanstieg um 133°C auf eine maximale Temperatur von 173°C gemessen. Die maximale Temperatur lag nach 3978 Sekunden vor, nachdem die Mischung auf 40 °C temperiert war. Zur Berechnung der Effizienz der Polymerisationsstabilisatoren in Bezug auf MEHQ entspricht der Wert von 3978 Sekunden einer Effizienz von 100 %.

Für eine Mischung mit 5-Methyl-2-phenyl-4H-pyrazol-3-on als Polymerisationsstabilisator wurde ein 66 Sekunden dauernder Temperaturanstieg um 119°C auf eine maximale Temperatur von 159 °C beobachtet. Die maximale Temperatur lag nach 1056 Sekunden vor, nachdem die Mischung auf 40°C temperiert war.

Im Vergleich zu MEHQ weist 5-Methyl-2-phenyl-4H-pyrazol-3-on als Polymerisationsstabilisator somit eine Effizienz von 27 Prozent auf.

Ergebnisse der Tests in Acrylsäure mit nicht erfindungsgemäßen Verbindungen:

| Struktur | IUPAC-Namen | Relative Stabilisierung von Acrylsäure unter Luft bezogen auf MeHQ (100%) | Relative Stabilisierung von Acrylsäure unter Stickstoff bezogen auf MeHQ (100%)) |
|---|---|---|---|
| 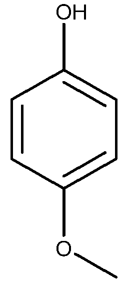 | 4-Methoxyphenol | 100 | 100 |
| 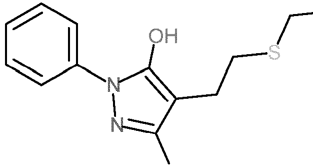 | 4-(2-Ethyl-sulfanylethyl)-5-methyl-2-phenyl-pyrazol-3-ol | 71 | 64 |
| 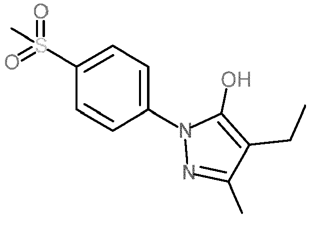 | 4-(2-Ethyl-sulfanylethyl)-5-methyl-2-(4-methylsul-fonylphenyl)pyra zol-3-ol | 68 | 50 |
| 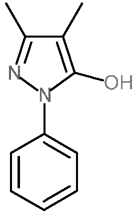 | 4,5-Dimethyl-2-phenyl-pyrazol-3-ol | 57 | 71 |
| 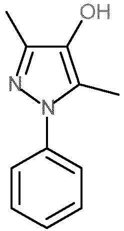 | 3,5-Dimethyl-1-phenyl-pyrazol-4-ol | 134 | 154 |
| 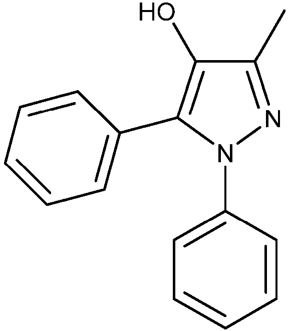 | 3-Methyl-1,5-diphenyl-pyrazol-4-ol | 97 | 161 |

Ergebnisse der Tests in Acrylsäure mit erfindungsgemäßen Verbindungen:

| Struktur | IUPAC-Name | Relative Stabilisierung von Acrylsäure unter Luft bezogen auf MeHQ (100%) | Relative Stabilisierung von Acrylsäure unter Stickstoff bezogen auf MeHQ (100%) |
|---|---|---|---|
| 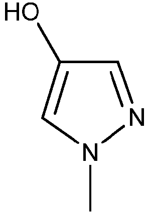 | 1-Methyl-pyrazol-4-ol | 93 | 53 |
| 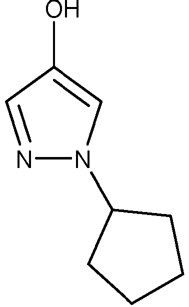 | 1-Cyclopentylpyrazol-4-ol | 144 | 104 |
| 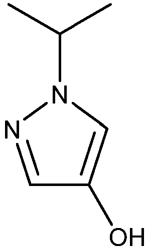 | 1-Isopropyl-pyrazol-4-ol | 89 | 80 |
| 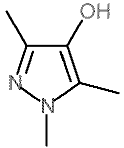 | 1,3,5-trimethyl-pyrazol-4-ol | 102 | 87 |
| 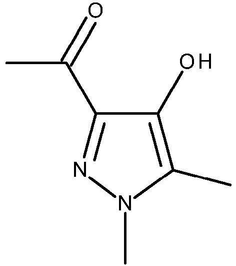 | 1-(4-hydroxy-1,5-dimethylpyrazol-3-yl)ethanone | 97% | 34% |
| 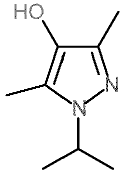 | 1-Isopropyl-3,5-dimethylpyrazol-4-ol | 238 | 122 |

### Polymerisationsversuche:

Unter einer Stickstoffatmosphäre wurden in einem Reaktionsgefäß 450 g deionisiertes Wasser vorgelegt. Die vorgelegte Reaktionsmischung wurde unter Rühren auf 95°C erhitzt. Nach Erreichen der Temperatur von 95°C erfolgte die Zuführung von drei Strömen unter Beibehaltung der Temperatur.
Strom 1: Zuführung über 5 h, 500 g Acrylsäure
Strom 2: Zuführung über 4,75 h, 15 g Natriumhypophosphite in 35 g deionisiertem Wasser
Strom 3: Zuführung über 5,25 h, 5 g Natriumpersulfat in 66,4 g deionisiertem Wasser Nach Zugabe der drei Ströme wurde die Reaktionsmischung für eine weitere Stunde bei 95°C gerührt. Die Reaktionsmischung wurde anschließend auf Raumtemperatur abgekühlt und mit 80 g deionisiertem Wasser versetzt.

In einem Vergleichsversuch war die Acrylsäure von Strom 1 mit 280 Gew.-ppm Edaravon (5-Methyl-2-phenyl-4H-pyrazol-3-on) bezogen auf die Masse der Acrylsäure stabilisiert. In einem weiteren Vergleichsversuch war die Acrylsäure von Strom 1 mit 200 Gew.-ppm MEHQ bezogen auf die Masse der Acrylsäure stabilisiert.

Die erhaltenen Polymere wurden mittels GPC analysiert (Kalibrierung mit Na-PAA-Standard, Elutionsmittel 0,01 mol/l Phosphatpuffer, pH=7,4 in dest. Wasser mit 0,01 M NaN3).

| | Mw (g/mol) |
|---|---|
| Polymer (Edaravon) | 9560 |
| Polymer (MEHQ) | 9400 |

### Tests in Acrylaten:

### Versuche zur Polymerisationsinhibierung unter Luft oder Stickstoff:

Für die Versuche zur Polymerisationsinhibierung unter Luft oder Stickstoff wurde entstabilisiertes Ethylacrylat verwendet. Hierfür wurde Ethylacrylat, das 10 bis 20 Gew.-ppm MEHQ bezogen auf die Menge an Ethylacrylat enthält drucklos über Aluminiumoxid Typ 90 aktiv neutral filtriert. Pro 100 ml stabilisiertem Ethylacrylat wurden 20 g Aluminiumoxid Typ 90 aktiv neutral verwendet. Nach der Filtration war mittels HPLC-Analytik kein MEHQ mehr im Ethylacrylat nachweisbar.

Für die Vergleichsversuche wurde eine 13 Gew.-ppm MEHQ enthaltende Ethylacrylatlösung hergestellt. Die Angabe Gew.-ppm bezieht sich auf die Masse des in der Lösung enthaltenen Ethylacrylats.

Für weitere Vergleichsversuche wurden Mischungen von Ethylacrylat mit Verbindungen die von den Verbindungen der allgemeinen Formel (II) unterschiedlich sind hergestellt, wobei die Molmenge der eingesetzten Verbindungen der Molmenge an MEHQ in den Vergleichsversuchen entspricht.

Für die erfindungsgemäßen Versuche wurden Mischungen von Ethylacrylat mit Verbindungen der allgemeinen Formel (II) hergestellt, wobei die Molmenge an Verbindungen der allgemeinen Formel (II) in den Mischungen der Molmenge an MEHQ in den Vergleichsversuchen entspricht.

Exemplarische Verdünnungsreihe:
Von einer Ethylacrylatlösung mit einem Gehalt von 500 Gew.-ppm MEHQ bezogen auf die Masse des in der Lösung enthaltenen Ethylacrylats wurde 1,0 g entnommen und mit Ethylacrylat auf einen Gehalt von 240 Gew.-ppm MEHQ bezogen auf die Masse des Ethylacrylats in der Lösung verdünnt.

Von der so erhaltenen Ethylacrylatlösung wurden 1,5 g entnommen und mit Ethylacrylat auf einen Gehalt von 80 Gew.-ppm MEHQ bezogen auf die Masse des Ethylacrylats in der Lösung verdünnt.

Von der so erhaltenen Ethylacrylatlösung wurden 1.5 g entnommen und mit Ethylacrylat auf einen Gehalt von 30 Gew.-ppm MEHQ bezogen auf die Masse des Ethylacrylats in der Lösung verdünnt.

Von der so erhaltenen Ethylacrylatlösung wurden 2,0 g entnommen und mit Ethylacrylat auf einen Gehalt von 13 Gew. ppm MEHQ bezogen auf die Masse des Ethylacrylats in der Lösung verdünnt.

Als Starterlösung wurde eine frisch angesetzte Toluol-Lösung von 2,2'-Azobis(4-methoxy-2,4-dimehtylvaleronitril) verwendet. Der Gehalt an 2,2'-Azobis(4-methoxy-2,4-dimehtylvaleronitril) in der Starterlösung beträgt 6420 Gew. ppm bezogen auf die Masse an Toluol in der Starterlösung.

### Tests zur Polymerisationsinhibierung unter Luft:

Unter Luft wurden 2.0 g Ethylacrylatlösung enthaltend 0,00021 mmol des jeweiligen Polymerisationsstabilisators vorgelegt. Die vorgelegte Ethylacrylatlösung wurde mit 0,17 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß.

Das Reaktionsgefäß enthaltend die Mischung wurde in einem auf 40°C erwärmten Heizblock platziert.

Nachdem die Mischung die Temperatur von 40°C erreicht hat wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 1200 Sekunden dauernder Temperaturanstieg um 8°C auf eine maximale Temperatur von 48°C gemessen. Die maximale Temperatur lag nach 29084 Sekunden vor, nachdem die Mischung auf 40°C temperiert war. Zur Berechnung der Effizienz der Polymerisationsstabilisatoren in Bezug auf MEHQ entspricht der Wert von 29084 Sekunden einer Effizienz von 100 %.

Für eine Mischung mit 5-Methyl-2-phenyl-4H-pyrazol-3-on als Polymerisationsstabilisator wurde ein 1200 Sekunden dauernder Temperaturanstieg um 6°C auf eine maximale Temperatur von 46°C beobachtet. Die maximale Temperatur lag nach 28470 Sekunden vor, nachdem die Mischung auf 40°C temperiert war.

Im Vergleich zu MEHQ weist 5-Methyl-2-phenyl-4H-pyrazol-3-on als Polymerisationsstabilisator somit eine Effizienz von 98 Prozent auf.

### Tests zur Polymerisationsinhibierung unter Stickstoff:

Unter Luft wurden 2.0 g Ethylacrylatlösung enthaltend 000021 mmol des jeweiligen Polymerisationsstabilisators vorgelegt. Die vorgelegte Ethylacrylatlösung wurde mit 0,17 g Starterlösung versetzt und homogenisiert. 1,9 ml der so erhaltenen Mischung wurden unter Luft in ein 4,5 ml Reaktionsgefäß gefüllt. Das Reaktionsgefäß wurde luftdicht verschlossen und mit einem Temperaturfühler versehen. Der Temperaturfühler ragte in die Mischung im Reaktionsgefäß. In das Reaktionsgefäß wurde oberhalb der vorgelegten Mischung Stickstoff unter Druckausgleich eingeleitet. Der Stickstoffstrom beträgt 3 bis 4 I/h. Die Einleitdauer beträgt 20 Sekunden. Der Stickstoff weist eine Reinheit von größer 99,9 % auf.

Das Reaktionsgefäß enthaltend die Mischung wurde in einem Heizblock platziert. Die Mischung wurde anschließend im Heizblock innerhalb von 4 Minuten auf 40°C erwärmt.

Nachdem die Mischung die Temperatur von 40°C erreicht hat wurde der Temperaturverlauf über die Zeit gemessen. Der Zeitraum von Erreichen der 40°C bis zum Erreichen der Maximaltemperatur wird als Bezugswert für die Inhibierungsleistung des jeweiligen Polymerisationsinhibitors herangezogen.

Für eine Mischung mit MEHQ als Polymerisationsstabilisator wurde ein 240 Sekunden dauernder Temperaturanstieg um 73°C auf eine maximale Temperatur von 113°C gemessen. Die maximale Temperatur lag nach 460 Sekunden vor, nachdem die Mischung auf 40°C temperiert war. Zur Berechnung der Effizienz der Polymerisationsstabilisatoren in Bezug auf MEHQ entspricht der Wert von 460 Sekunden einer Effizienz von 100 %.

Für eine Mischung mit 5-Methyl-2-phenyl-4H-pyrazol-3-on als Polymerisationsstabilisator wurde ein 240 Sekunden dauernder Temperaturanstieg um 80°C auf eine maximale Temperatur von 120°C beobachtet. Die maximale Temperatur lag nach 426 Sekunden vor, nachdem die Mischung auf 40°C temperiert war.

Im Vergleich zu MEHQ weist 5-Methyl-2-phenyl-4H-pyrazol-3-on als Polymerisationsstabilisator somit eine Effizienz von 93 Prozent auf.

Ergebnisse der Tests in Ethylacrylat mit erfindungsgemäßen und nicht erfindungsgemäßen Verbindungen:

| Struktur | IUPAC-Name | Relative Stabilisierung von Ethylacrylat unter Luft bezogen auf MeHQ (100%) | Relative Stabilisierung von Ethylacrylat unter Stickstoff bezogen auf MeHQ (100%) |
|---|---|---|---|
| 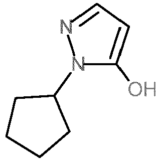 | 2-Cyclo-pentylpyrazol-3-ol | 97 | 45 |
| 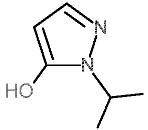 | 2-Isopropyl-pyrazol-3-ol | 95 | 69 |
| 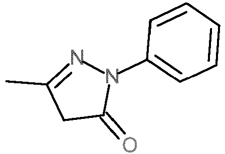 | 5-Methyl-2-phenyl-4H-pyrazol-3-one | 86 | 45 |
| 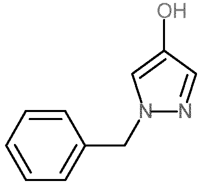 | 1-benzylpyrazol-4-ol | 107 | 58 |
| 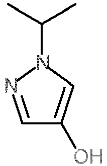 | 1-Isopropylpyrazol-4-ol | 106 | 101 |
| 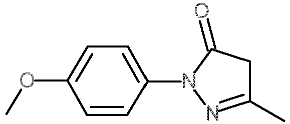 | 2-(4-Methoxyphenyl)-5-methyl-4H-pyrazol-3-one | 89 | 46 |
| 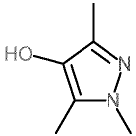 | 1,3,5-Trimethylpyrazol-4-ol | 117 | 97 |
| 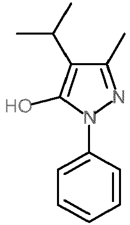 | 4-Isopropyl-5-methyl-2-phenyl-pyrazol-3-ol | 151 | 63 |
| 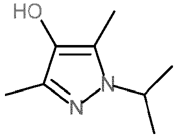 | 1-Isopropyl-3,5-dimethyl-pyrazol-4-ol | 122 | 86 |

### Polymerisationsversuche mit Acrylaten:

### Homopolymerisation von n-Butylacrylat:

### Vergleichsversuch:

Unter einer Stickstoffatmosphäre wurden in einem Reaktionsgefäß 210 g n-Butylacetat, 14,7 g n-Butylacrylat (stabilisiert mit 15 Gew. ppm MEHQ bezogen auf die Menge des n-Butylacrylats) und 0,08 g Azobisisobutyronitril (AIBN) vorgelegt. Die vorgelegte Reaktionsmischung wurde unter Rühren auf 80°C erhitzt. Nach 15 Minuten nach Erreichen der Temperatur von 80°C erfolgte die Zuführung von zwei Strömen unter Beibehaltung der Temperatur.
Strom 1: Zuführung über 2 h, 195,3 g n- Butylacrylat (stabilisiert mit 15 Gew. ppm MEHQ bezogen auf die Menge des n-Butylacrylats) und 100,5 g n-Butylacetat
Strom 2: Zuführung über 3 h, 0,98 g AIBN und 74,1 g n-Butylacetat

Die Reaktionsmischung wurde nach Zugabe der Ströme für 2h bei 80°C gerührt und anschließend auf 25°C abgekühlt.

Vergleichsversuch: Unter einer Stickstoffatmosphäre wurden in einem Reaktionsgefäß 210 g n-Butylacetat, 14,7 g n-Butylacrylat (stabilisiert mit 21 Gew.-ppm 5-Methyl-2-phenyl-4H-pyrazol-3-on bezogen auf die Menge des n-Butylacrylats) und 0,08 g AIBN vorgelegt. Die vorgelegte Reaktionsmischung wurde unter Rühren auf 80°C erhitzt. Nach 15 Minuten nach Erreichen der Temperatur von 80°C erfolgte die Zuführung von zwei Strömen unter Beibehaltung der Temperatur.
Strom 1: Zuführung über 2 h, 195,3 g n- Butylacrylat (stabilisiert mit 21 Gew.-ppm 5-Methyl-2-phenyl-4H-pyrazol-3-on bezogen auf die Menge des n-Butylacrylats) und 100,5 g n-Butylacetat
Strom 2: Zuführung über 3 h, 0,98 g AIBN und 74,1 g n-Butylacetat

Die Reaktionsmischung wurde nach Zugabe der Ströme für 2h bei 80°C gerührt und anschließend auf Raumtemperatur abgekühlt.

Die erhaltenen Polymere wurden mittels GPC analysiert (Kalibrierung Polystyrolstandard, Elutionsmittel: THF + 0,1 Vol. % Trifluoressigsäure).

| | Mw (g/mol) |
|---|---|
| Polymer (Vergleichsversuch) | 164000 |
| Polymer (MEHQ) | 163000 |

Emulsionspolymerisation von n-Butylacrylat, Styrol und Methacrylsäure:

### Vergleichsversuch:

Unter einer Stickstoffatmosphäre wurden in einem Reaktionsgefäß 250 g VE Wasser und 4 g Kaliumperoxodisulfat vorgelegt. Das vorgelegte Reaktionsgemisch wurde unter Rühren auf 60°C erwärmt. Bei 60°C wurden unter Rühren 8 g von Zulauf 1 dem Reaktionsgemisch zugeführt. 5 Minuten nach Zugabe wurden bei 60°C und unter Rühren der Rest von Zulauf 1 und Zulauf 2 dem Reaktionsgemisch zugeführt. Der Rest von Zulauf 1 wurde über eine Zeit von 160 Minuten, der Zulauf 2 wurde über eine Zeit von 150 Minuten dem Reaktionsgemisch zugeführt.

| | |
|---|---|
| Zulauf 1: | 4 g Natriumhydrogensulfit, 100 g VE Wasser |
| Zulauf 2: | 400 g Styrol (stabilisiert mit 20 Gew. ppm MEHQ bezogen auf die Menge Styrol), 400 g n-Butylacrylat (stabilisiert mit 15 Gew. ppm MEHQ bezogen auf die Menge n-Butylacrylat), 15 g Methacrylsäure (stabilisiert mit 200 Gew. ppm MEHQ bezogen auf die Menge Methacrylsäure), 20 g Disponil FES 147 und 450 g VE Wasser. |

Nach Beendigung der Zuführung von Zulauf 1 wurde die Temperatur des Reaktionsgemisches auf 65°C erhöht und das Reaktionsgemisch für 1 h bei dieser Temperatur gehalten. Die erhaltene Dispersion wurde auf Umgebungstemperatur abgekühlt, mit wässriger Ammoniaklösung (25 Gew. %) auf pH 7 eingestellt und durch ein Sieb filtriert.

### Vergleichsversuch 2:

Unter einer Stickstoffatmosphäre wurden in einem Reaktionsgefäß 250 g VE Wasser und 4 g Kaliumperoxodisulfat vorgelegt. Das vorgelegte Reaktionsgemisch wurde unter Rühren auf 60°C erwärmt. Bei 60°C wurden unter Rühren 8 g von Zulauf 1 dem Reaktionsgemisch zugeführt. 5 Minuten nach Zugabe wurden bei 60°C und unter Rühren der Rest von Zulauf 1 und Zulauf 2 dem Reaktionsgemisch zugeführt. Der Rest von Zulauf 1 wurde über eine Zeit von 160 Minuten, der Zulauf 2 wurde über eine Zeit von 150 Minuten dem Reaktionsgemisch zugeführt.

| | |
|---|---|
| Zulauf 1: | 4 g Natriumhydrogensulfit, 100 g VE Wasser |
| Zulauf 2: | 400 g Styrol (stabilisiert mit 28 Gew. ppm Edaravon bezogen auf die Menge Styrol), 400 g n-Butylacrylat (stabilisiert mit 21 Gew. ppm Edaravon bezogen auf die Menge n-Butylacrylat), 15 g Methacrylsäure (stabilisiert mit 280 Gew. ppm Edaravon bezogen auf die Menge Methacrylsäure), 20 g Disponil FES 147 und 450 g VE Wasser. |

Nach Beendigung der Zuführung von Zulauf 1 wurde die Temperatur des Reaktionsgemisches auf 65°C erhöht und das Reaktionsgemisch für 1 h bei dieser Temperatur gehalten. Die erhaltene Dispersion wurde auf Umgebungstemperatur abgekühlt, mit wässriger Ammoniaklösung (25 Gew. %) auf pH 7 eingestellt und durch ein Sieb filtriert.

| | Mittlerer Teilchendurchmesser (nm) | Glastemperatur (°C) | Mw (g/mol) | Koagulat (Gew.%) | Restmonomerengehalt (Gew. ppm) |
|---|---|---|---|---|---|
| Vergleichsversuch | 163 | 19 | 820000 | 0,25 | n-Butylacrylat: 300 |
| | | | | | Styrol: < 20 |
| Vergleichsversuch 2 | 168 | 21 | 850000 | 0,4 | n-Butylacrylat: 500 |
| | | | | | Styrol: < 20 |

Der mittlere Teilchendurchmesser (z-Average) wurde wie folgt bestimmt:
Die Proben wurden mit einem Zetasizer Nano der Firma Malvern analysiert. Sie wurden mit Milliporewasser (pH4) auf eine Messkonzentration von 0,0005% verdünnt und dann 5µm filtriert bestimmt. Die Messungen erfolgten bei 25°C.

Die Glastemperatur wurde wie folgt ermittelt:
Die Polymerdispersionen wurden bei 80°C/Vakuum getrocknet. Die Glastemperatur wurde mittels DSC bestimmt. Vor der Messung wurden die Proben von 140°C aus schnell abgekühlt und anschließend mit 20 K/min von -50°C bis 140°C vermessen.

Die Molmassenbestimmung erfolgte mittels GPC (Kalibrierung mit Polystyrol-Standard, Elutionsmittel: THF + 0.1 Vol. % Trifluoressigsäure).

Der Restmonomergehalt wurde mittels Head-Space GC bestimmt.

Die Koagulatmenge (aggregierte Polymerkolloide in der Apparatur und im Filtrationsrückstand) wurde gewogen.

## Patentansprüche

1. Mischung enthaltend eine oder mehrere Verbindungen der allgemeinen Formel (II) 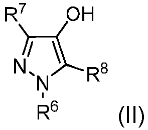 wobei
R⁶ H oder C₁- bis C₆-Alkyl ist, wobei C₁- bis C₆-Alkyl gerade oder verzweigte C₁-bis C₆-Alkylgruppen oder cyclische C₃- bis C₆-Alkylgruppen umfasst,
R⁷ H, C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst, oder Alkanoyl mit C₁- bis C₆-Alkyl ist, wobei C₁- bis C₆-Alkyl gerade oder verzweigte C₁- bis C₆-Alkylgruppen oder cyclische C₃- bis C₆-Alkylgruppen umfasst, und
R⁸ H oder C₁- bis C₆-Alkyl ist, wobei C₁- bis C₆-Alkyl gerade oder verzweigte C₁-bis C₆-Alkylgruppen oder cyclische C₃- bis C₆-Alkylgruppen umfasst,
und (Meth)acrylsäure und/oder (Meth)acrylsäureester mit C₁- bis C₈-Alkyl, wobei C₁- bis C₈-Alkyl gerade oder verzweigte C₁- bis C₈-Alkylgruppen oder cyclische C₃- bis C₈-Alkylgruppen umfasst.

2. Mischung nach Anspruch 1, wobei es sich bei (Meth)acrylsäureester mit C₁- bis C₈-Alkyl um Methyl(meth)acrylat, Ethyl(meth)acrylat, n-Butyl(meth)acrylat, 2-Ethylhexyl(meth)acrylat oder um eine Mischung aus zwei oder mehr der zuvor genannten (Meth)acrylsäureester handelt.

3. Mischung nach Anspruch 1 oder 2, wobei die Gesamtmenge der Verbindungen der allgemeinen Formel (II) in der Mischung 0,1 bis 1000 Gew.-ppm beträgt, bezogen auf das Gesamtgewicht der in der Mischung enthaltenen Menge an (Meth)acrylsäure und (Meth)acrylsäureester mit C₁- bis C₈-Alkyl.

4. Mischung nach Anspruch 1 oder 2, wobei die Gesamtmenge der Verbindungen der allgemeinen Formel (II) in der Mischung 1 bis 900 Gew.-ppm beträgt, jeweils bezogen auf das Gesamtgewicht der in der Mischung enthaltenen Menge an (Meth)acrylsäure und (Meth)acrylsäureester mit C₁- bis C₈-Alkyl.

5. Mischung nach Anspruch 1 oder 2, wobei die Gesamtmenge der Verbindungen der allgemeinen Formel (II) in der Mischung 10 bis 500 Gew.-ppm beträgt, jeweils bezogen auf das Gesamtgewicht der in der Mischung enthaltenen Menge an (Meth)acrylsäure und (Meth)acrylsäureester mit C₁- bis C₈-Alkyl.

6. Mischung nach einem der Ansprüche 1 bis 5, wobei die Gesamtmenge an (Meth)acrylsäure und (Meth)acrylsäureester mit C₁- bis C₈-Alkyl in der Mischung mindestens 5 Gew.-% bezogen auf das Gesamtgewicht der Mischung beträgt.

7. Mischung nach einer der Ansprüche 1 bis 5, wobei die Gesamtmenge an (Meth)acrylsäure und (Meth)acrylsäureester mit C₁- bis C₈-Alkyl in der Mischung mindestens 80 Gew.-% beträgt, bezogen auf das Gesamtgewicht der Mischung.

8. Mischung nach einer der Ansprüche 1 bis 5, wobei die Gesamtmenge an (Meth)acrylsäure und (Meth)acrylsäureester mit C₁- bis C₈-Alkyl in der Mischung mindestens 95 Gew.-% beträgt, bezogen auf das Gesamtgewicht der Mischung.

9. Mischung nach einem der Ansprüche 1 oder 3 bis 8, wobei die Mischung (Meth)acrylsäure enthält und wobei die Mischung eine oder mehrere der nachfolgenden Komponenten enthält:
a. Wasser 20 bis 15000 Gew.-ppm bezogen auf das Gesamtgewicht der in der Mischung enthaltenen (Meth)acrylsäure,
b. Di(meth)acrylsäure 100 bis 20000 Gew.-ppm bezogen auf das Gesamtgewicht der in der Mischung enthaltenen (Meth)acrylsäure,
c. Essigsäure 100 bis 2000 Gew.-ppm bezogen auf das Gesamtgewicht der in der Mischung enthaltenen (Meth)acrylsäure,
d. Propionsäure 100 bis 2000 Gew.-ppm bezogen auf das Gesamtgewicht der n der Mischung enthaltenen (Meth)acrylsäure,
e. ein oder mehrere Aldehyde wie Acetaldehyd, Acrolein, Propionaldehyd, Furfural-2, Furfural-3, Cumaron oder Benzaldehyd, wobei die Menge pro Aldehyd 0 bis 1 Gew.-ppm bezogen auf das Gesamtgewicht der in der Mischung enthaltenen (Meth)acrylsäure beträgt,
f. Protoanemonin 0 bis 2 Gew.-ppm bezogen auf das Gesamtgewicht der in der Mischung enthaltenen (Meth)acrylsäure,
g. Maleinsäure 0 bis 1 Gew.-ppm bezogen auf das Gesamtgewicht der in der Mischung enthaltenen (Meth)acrylsäure,
h. Maleinsäureanhydrid 0 bis 1 Gew.-ppm bezogen auf das Gesamtgewicht der in der Mischung enthaltenen (Meth)acrylsäure,
i. Allylacrylat 0 bis 30 Gew.-ppm, bevorzugt 0 bis 1 Gew.-ppm bezogen auf das Gesamtgewicht der in der Mischung enthaltenen (Meth)acrylsäure.

10. Mischung nach einem der Ansprüche 2 bis 8, wobei die Mischung einen oder mehrere (Meth)acrylsäureester gemäß Anspruch 2 enthält und wobei die Mischung eine oder mehrere der nachfolgenden Komponenten enthält:
a. Wasser 1 bis 2000 Gew.-ppm, bevorzugt 1 bis 500 Gew.-ppm jeweils bezogen auf das Gesamtgewicht der in der Mischung enthaltenen (Meth)acrylsäureester gemäß Anspruch 2,
b. (Meth)acrylsäure 1 bis 2000 Gew.-ppm, bevorzugt 1 bis 100 Gew.-ppm jeweils bezogen auf das Gesamtgewicht der in der Mischung enthaltenen (Meth)acrylsäureester gemäß Anspruch 2,
c. Alkohole der jeweiligen (Meth)acrylsäureester 0,01 bis 2000 Gew.-ppm, bevorzugt 0,1 bis 1000 Gew.-ppm jeweils bezogen auf das Gesamtgewicht der in der Mischung enthaltenen (Meth)acrylsäureester gemäß Anspruch 2,
d. Propionsäureester mit den Alkoholen der jeweiligen (Meth)acrylsäureester 0,1 bis 2000 Gew.-ppm, bevorzugt 10 bis 1000 Gew.-ppm jeweils bezogen auf das Gesamtgewicht der in der Mischung enthaltenen (Meth)acrylsäureester gemäß Anspruch 2,
e. Essigsäureester mit den Alkoholen der jeweiligen (Meth)acrylsäureester 1 bis 2000 Gew.-ppm, bevorzugt 5 bis 2000 Gew.-ppm jeweils bezogen auf das Gesamtgewicht der in der Mischung enthaltenen (Meth)acrylsäureester gemäß Anspruch 2,
f. Ether der jeweiligen (Meth)acrylsäureester-Alkohole 0 bis 2000 Gew.-ppm bezogen auf das Gesamtgewicht der in der Mischung enthaltenen (Meth)acrylsäureester gemäß Anspruch 2.

11. Mischung nach einem der Ansprüche 1 bis 10, wobei die Mischung unter einer sauerstoffhaltigen Atomsphäre gelagert und/oder transportiert wird.

12. Mischung nach einem der Ansprüche 1 bis 11, wobei die Mischung einen Costabilisator enthält.

13. Verwendung einer oder mehrerer Verbindungen der allgemeinen Formel (II) wie in Anspruch 1 definiert zur Lager- und/oder Transportstabilisierung von (Meth)acrylsäure und/oder (Meth)acrylsäureester mit C₁- bis C₈-Alkyl.

14. Verfahren zur Polymerisation von (Meth)acrylsäure und/oder (Meth)acrylsäureester mit C₁- bis C₈-Alkyl in Gegenwart einer oder mehrerer Verbindungen der allgemeinen Formel (II) wie in Anspruch 1 definiert, wobei die Reaktionsmischung zu 0 bis 50% mit Sauerstoff gesättigt ist, wobei der Grad der Sauerstoffsättigung sich auf den Grad der Sauerstoffsättigung bezieht der im Gleichgewicht erreicht wird, wenn die vorliegende Mischung unter Standardbedingungen unter einer sauerstoffhaltigen Atmosphäre mit einem Sauerstoffanteil von 5 bis 30 vol.% gelagert und/oder transportiert wird.

15. Verfahren zur Stabilisierung von (Meth)acrylsäure und/oder (Meth)acrylsäureester mit C₁- bis C₈-Alkyl unter Verwendung einer oder mehrerer Verbindungen der allgemeinen Formel (II) wie in Anspruch 1 definiert.

## Claims

1. A mixture comprising one or more compounds of the general formula (II) 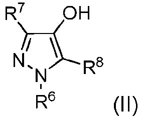 where
R⁶ is H or C₁ to C₆ alkyl, where C₁ to C₆ alkyl comprises straight-chain or branched C₁ to C₆ alkyl groups or cyclic C₃ to C₆ alkyl groups,
R⁷ is H, C₁ to C₆ alkyl, where C₁ to C₆ alkyl comprises straight-chain or branched C₁ to C₆ alkyl groups or cyclic C₃ to C₆ alkyl groups, or is alkanoyl with C₁ to C₆ alkyl, where C₁ to C₆ alkyl comprises straight-chain or branched C₁ to C₆ alkyl groups or cyclic C₃ to C₆ alkyl groups, and R⁸ is H or C₁ to C₆ alkyl, where C₁ to C₆ alkyl comprises straight-chain or branched C₁ to C₆ alkyl groups or cyclic C₃ to C₆ alkyl groups,
and (meth)acrylic acid and/or (meth)acrylic esters with C₁ to C₈ alkyl, where C₁ to C₈ alkyl comprises straight-chain or branched C₁ to C₈ alkyl groups or cyclic C₃ to C₈ alkyl groups.

2. The mixture according to claim 1, wherein (meth)acrylic esters with C₁ to C₈ alkyl are methyl (meth)acrylate, ethyl (meth)acrylate, n-butyl (meth)acrylate, 2-ethylhexyl (meth)acrylate or a mixture of two or more of the above-mentioned (meth)acrylic esters.

3. The mixture according to claim 1 or 2, wherein the total amount of compounds of the general formula (II) in the mixture is 0.1 to 1000 ppm by weight, based on the total weight of the amount of (meth)acrylic acid and (meth)acrylic esters with C₁ to C₈ alkyl that is present in the mixture.

4. The mixture according to claim 1 or 2, wherein the total amount of compounds of the general formula (II) in the mixture is 1 to 900 ppm by weight, in each case based on the total weight of the amount of (meth)acrylic acid and (meth)acrylic esters with C₁ to C₈ alkyl that is present in the mixture.

5. The mixture according to claim 1 or 2, wherein the total amount of compounds of the general formula (II) in the mixture is 10 to 500 ppm by weight, in each case based on the total weight of the amount of (meth)acrylic acid and (meth)acrylic esters with C₁ to C₈ alkyl that is present in the mixture.

6. The mixture according to any of claims 1 to 5, wherein the total amount of (meth)acrylic acid and (meth)acrylic esters with C₁ to C₈ alkyl in the mixture is at least 5% by weight, based on the total weight of the mixture.

7. The mixture according to any of claims 1 to 5, wherein the total amount of (meth)acrylic acid and (meth)acrylic esters with C₁ to C₈ alkyl in the mixture is at least 80% by weight, based on the total weight of the mixture.

8. The mixture according to any of claims 1 to 5, wherein the total amount of (meth)acrylic acid and (meth)acrylic esters with C₁ to C₈ alkyl in the mixture is at least 95% by weight, based on the total weight of the mixture.

9. The mixture according to any of claims 1 or 3 to 8, wherein the mixture comprises (meth)acrylic acid and wherein the mixture comprises one or more of the following components:
a. water: 20 to 15 000 ppm by weight based on the total weight of the (meth)acrylic acid present in the mixture,
b. di(meth)acrylic acid: 100 to 20 000 ppm by weight based on the total weight of the (meth)acrylic acid present in the mixture,
c. acetic acid: 100 to 2000 ppm by weight based on the total weight of the (meth)acrylic acid present in the mixture,
d. propionic acid: 100 to 2000 ppm by weight based on the total weight of the (meth)acrylic acid present in the mixture,
e. one or more aldehydes such as acetaldehyde, acrolein, propionaldehyde, 2-furfural, 3-furfural, coumarone or benzaldehyde, the amount of each aldehyde being 0 to 1 ppm by weight based on the total weight of the (meth)acrylic acid present in the mixture,
f. protoanemonin: 0 to 2 ppm by weight based on the total weight of the (meth)acrylic acid present in the mixture,
g. maleic acid: 0 to 1 ppm by weight based on the total weight of the (meth)acrylic acid present in the mixture,
h. maleic anhydride: 0 to 1 ppm by weight based on the total weight of the (meth)acrylic acid present in the mixture,
i. allyl acrylate: 0 to 30 ppm by weight, preferably 0 to 1 ppm by weight, based on the total weight of the (meth)acrylic acid present in the mixture.

10. The mixture according to any of claims 2 to 8, wherein the mixture comprises one or more (meth)acrylic esters according to claim 2 and wherein the mixture comprises one or more of the following components:
a. water: 1 to 2000 ppm by weight, preferably 1 to 500 ppm by weight, in each case based on the total weight of the (meth)acrylic esters according to claim 2 present in the mixture,
b. (meth)acrylic acid: 1 to 2000 ppm by weight, preferably 1 to 100 ppm by weight, in each case based on the total weight of the (meth)acrylic esters according to claim 2 present in the mixture,
c. alcohols of the respective (meth)acrylic esters: 0.01 to 2000 ppm by weight, preferably 0.1 to 1000 ppm by weight, in each case based on the total weight of the (meth)acrylic esters according to claim 2 present in the mixture,
d. propionic esters with the alcohols of the respective (meth)acrylic esters: 0.1 to 2000 ppm by weight, preferably 10 to 1000 ppm by weight, in each case based on the total weight of the (meth)acrylic esters according to claim 2 present in the mixture,
e. acetic esters with the alcohols of the respective (meth) acrylic esters: 1 to 2000 ppm by weight, preferably 5 to 2000 ppm by weight, in each case based on the total weight of the (meth)acrylic esters according to claim 2 present in the mixture,
f. ethers of the respective (meth)acrylic ester alcohols: 0 to 2000 ppm by weight, based on the total weight of the (meth)acrylic esters according to claim 2 present in the mixture.

11. The mixture according to any of claims 1 to 10, wherein the mixture is stored and/or transported under an oxygen-containing atmosphere.

12. The mixture according to any of claims 1 to 11, wherein the mixture comprises a co-stabilizer.

13. The use of one or more compounds of the general formula (II) as defined in claim 1 for the storage stabilization and/or transport stabilization of (meth)acrylic acid and/or (meth)acrylic esters with C₁ to C₈ alkyl.

14. A method for polymerizing (meth)acrylic acid and/or (meth)acrylic esters with C₁ to C₈ alkyl in the presence of one or more compounds of the general formula (II) as defined in claim 1, wherein the reaction mixture is 0% to 50% saturated with oxygen, the degree of oxygen saturation referring to the degree of oxygen saturation that is achieved at equilibrium when the present mixture is stored and/or transported under standard conditions under an oxygen-containing atmosphere having an oxygen content of 5 to 30 vol%.

15. A method for stabilizing (meth)acrylic acid and/or (meth) acrylic esters with C₁ to C₈ alkyl using one or more compounds of the general formula (II) as defined in claim 1.

## Revendications

1. Mélange contenant un ou plusieurs composés de formule générale (II) 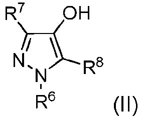 dans laquelle
R⁶ est H ou alkyle en C₁-C₆, alkyle en C₁-C₆ comprenant des groupes alkyle en C₁-C₆ à chaîne droite ou ramifiés ou des groupes alkyle en C₃-C₆ cycliques,
R⁷ est H, alkyle en C₁-C₆, alkyle en C₁-C₆ comprenant des groupes alkyle en C₁-C₆ à chaîne droite ou ramifiés ou des groupes alkyle en C₃-C₆ cycliques, ou alcanoyle comportant un alkyle en C₁-C₆, alkyle en C₁-C₆ comprenant des groupes alkyle en C₁-C₆ à chaîne droite ou ramifiés ou des groupes alkyle en C₃-C₆ cycliques, et
R⁸ est H ou alkyle en C₁-C₆, alkyle en C₁-C₆ comprenant des groupes alkyle en C₁-C₆ à chaîne droite ou ramifiés ou des groupes alkyle en C₃-C₆ cycliques,
et de l'acide (méth)acrylique et/ou des esters d'acide (méth)acrylique comportant un alkyle en C₁-C₈, alkyle en C₁-C₈ comprenant des groupes alkyle en C₁-C₈ à chaîne droite ou ramifiés ou des groupes alkyle en C₃-C₈ cycliques.

2. Mélange selon la revendication 1, dans lequel les esters d'acide (méth)acrylique comportant un alkyle en C₁-C₈ consistent en du (méth)acrylate de méthyle, du (méth)acrylate d'éthyle, du (méth)acrylate de n-butyle, du (méth)acrylate de 2-éthylhexyle ou un mélange de deux ou plus de deux esters d'acide (méth)acrylique mentionnés précédemment.

3. Mélange selon la revendication 1 ou 2, dans lequel la quantité totale des composés de formule générale (II) dans le mélange vaut de 0,1 à 1 000 ppm en poids, par rapport au poids total de la quantité d'acide (méth)acrylique et d'esters d'acide (méth)acrylique comportant un alkyle en C₁-C₈ contenue dans le mélange.

4. Mélange selon la revendication 1 ou 2, dans lequel la quantité totale des composés de formule générale (II) dans le mélange vaut de 1 à 900 ppm en poids, chaque fois par rapport au poids total de la quantité d'acide (méth)acrylique et d'esters d'acide (méth)acrylique comportant un alkyle en C₁-C₈ contenue dans le mélange.

5. Mélange selon la revendication 1 ou 2, dans lequel la quantité totale des composés de formule générale (II) dans le mélange vaut de 10 à 500 ppm en poids, chaque fois par rapport au poids total de la quantité d'acide (méth)acrylique et d'esters d'acide (méth)acrylique comportant un alkyle en C₁-C₈ contenue dans le mélange.

6. Mélange selon l'une quelconque des revendications 1 à 5, dans lequel la quantité totale d'acide (méth)acrylique et d'esters d'acide (méth)acrylique comportant un alkyle en C₁-C₈ dans le mélange vaut au moins 5 % en poids par rapport au poids total du mélange.

7. Mélange selon l'une quelconque des revendications 1 à 5, dans lequel la quantité totale d'acide (méth)acrylique et d'esters d'acide (méth)acrylique comportant un alkyle en C₁-C₈ dans le mélange vaut au moins 80 % en poids, par rapport au poids total du mélange.

8. Mélange selon l'une quelconque des revendications 1 à 5, dans lequel la quantité totale d'acide (méth)acrylique et d'esters d'acide (méth)acrylique comportant un alkyle en C₁-C₈ dans le mélange vaut au moins 95 % en poids, par rapport au poids total du mélange.

9. Mélange selon l'une quelconque des revendications 1 ou 3 à 8, dans lequel le mélange contient de l'acide (méth)acrylique et dans lequel le mélange contient un ou plusieurs des composants suivants :
a. eau 20 à 15 000 ppm en poids par rapport au poids total de l'acide (méth)acrylique contenu dans le mélange,
b. acide di(méth) acrylique 100 à 20 000 ppm en poids par rapport au poids total de l'acide (méth)acrylique contenu dans le mélange,
c. acide acétique 100 à 2 000 ppm en poids par rapport au poids total de l'acide (méth)acrylique contenu dans le mélange,
d. acide propionique 100 à 2 000 ppm en poids par rapport au poids total de l'acide (méth)acrylique contenu dans le mélange,
e. un ou plusieurs aldéhydes tels que l'acétaldéhyde, l'acroléine, le propionaldéhyde, le furfural-2, le furfural-3, la coumarone ou le benzaldéhyde, la quantité par aldéhyde valant de 0 à 1 ppm en poids par rapport au poids total de l'acide (méth)acrylique contenu dans le mélange,
f. proto-anémonine 0 à 2 ppm en poids par rapport au poids total de l'acide (méth)acrylique contenu dans le mélange,
g. acide maléique 0 à 1 ppm en poids par rapport au poids total de l'acide (méth)acrylique contenu dans le mélange,
h. anhydride maléique 0 à 1 ppm en poids par rapport au poids total de l'acide (méth)acrylique contenu dans le mélange,
i. acrylate d'allyle 0 à 30 ppm en poids, de préférence 0 à 1 ppm en poids par rapport au poids total de l'acide (méth)acrylique contenu dans le mélange.

10. Mélange selon l'une quelconque des revendications 2 à 8, dans lequel le mélange contient un ou plusieurs esters d'acide (méth)acrylique selon la revendication 2 et dans lequel le mélange contient un ou plusieurs des composants suivants :
a. eau 1 à 2 000 ppm en poids, de préférence 1 à 500 ppm en poids, chaque fois par rapport au poids total des esters d'acide (méth)acrylique selon la revendication 2 contenus dans le mélange
b. acide (méth)acrylique 1 à 2 000 ppm en poids, de préférence 1 à 100 ppm en poids, chaque fois par rapport au poids total des esters d'acide (méth)acrylique selon la revendication 2 contenus dans le mélange ,
c. alcools des esters d'acide (méth)acrylique respectifs 0,01 à 2 000 ppm en poids, de préférence 0,1 à 1 000 ppm en poids, chaque fois par rapport au poids total des esters d'acide (méth)acrylique selon la revendication 2 contenus dans le mélange,
d. esters d'acide propionique avec les alcools des esters d'acide (méth)acrylique respectifs 0,1 à 2 000 ppm en poids, de préférence 10 à 1 000 ppm en poids, chaque fois par rapport au poids total des esters d'acide (méth)acrylique selon la revendication 2 contenus dans le mélange,
e. esters d'acide acétique avec les alcools des esters d'acide (méth)acrylique respectifs 1 à 2 000 ppm en poids, de préférence 5 à 2 000 ppm en poids, chaque fois par rapport au poids total des esters d'acide (méth)acrylique selon la revendication 2 contenus dans le mélange,
f. éthers des alcools d'esters d'acide (méth)acrylique respectifs 0 à 2 000 ppm en poids par rapport au poids total des esters d'acide (méth)acrylique selon la revendication 2 contenus dans le mélange.

11. Mélange selon l'une quelconque des revendications 1 à 10, le mélange étant stocké et/ou transporté sous une atmosphère contenant de l'oxygène.

12. Mélange selon l'une quelconque des revendications 1 à 11, le mélange contenant un co-stabilisant.

13. Utilisation d'un ou de plusieurs composés de formule générale (II) telle que définie dans la revendication 1 pour la stabilisation au stockage et/ou au transport d'acide (méth)acrylique et/ou d'esters d'acide (méth)acrylique comportant un alkyle en C₁-C₈.

14. Procédé pour la polymérisation d'acide (méth)acrylique et/ou d'esters d'acide (méth)acrylique comportant un alkyle en C₁-C₈ en présence d'un ou de plusieurs composés de formule générale (II) telle que définie dans la revendication 1, dans lequel le mélange réactionnel est saturé à 0 à 50 % avec de l'oxygène, le degré de saturation en oxygène se rapportant au degré de saturation en oxygène qui est atteint en équilibre lorsque le mélange présent est stocké et/ou transporté dans des conditions standard sous une atmosphère contenant de l'oxygène ayant une teneur en oxygène de 5 à 30 % en volume.

15. Procédé pour la stabilisation d'acide (méth)acrylique et/ou d'esters d'acide (méth)acrylique comportant un alkyle en C₁-C₈, avec utilisation d'un ou de plusieurs composés de formule générale (II) telle que définie dans la revendication 1.
